# EUROPEAN PATENT APPLICATION

(11) **EP 1 142 881 A1**
(43) Date of publication of application: **10.10.2001**
(21) Application number: 00900393.0
(22) Date of filing: 14.01.2000
(51) Int. Cl.: C07D 239/88, C07D 495/04, C07D 491/052, A61K 31/517, A61K 31/519, A61P 25/00, A61P 29/00

(54) **POLY(ADP-RIBOSE) POLYMERASE INHIBITORS CONSISTING OF PYRIMIDINE DERIVATIVES**

(30) Priority: 14.01.1999 JP 844699
(71) Applicant: Meiji Seika Kaisha, Ltd., Tokyo 104-8002 (JP)
(72) Inventor: HASEGAWA, Toshifumi, Meiji Seika Kaisha, Ltd., Yokohama-shi, Kanagawa 222-8567 (JP); NAKAJIMA, Hidemitsu, Meiji Seika Kaisha, Ltd., Yokohama-shi, Kanagawa 222-8567 (JP); KUBOTA, Dai, Meiji Seika Kaisha, Ltd., Yokohama-shi, Kanagawa 222-8567 (JP); OKUMA, Kunihiro, Meiji Seika Kaisha, Ltd., Yokohama-shi, Kanagawa 222-8567 (JP)
(74) Representative: Sternagel, Fleischer, Godemeyer & Partner Patentanwälte
(86) International application number: JP0000153
(87) International publication number: WO0042025

(57) **Abstract**

A medicament for therapeutic and/or preventive treatment of a brain disease, which comprises a compound represented by the following general formula (I) or a pharmaceutically acceptable salt thereof as an active ingredient: 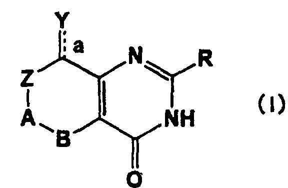 wherein R represents hydrogen atom, a C₁-C₈ alkyl group, a substituted C₁-C₈ alkyl group, an aryl group, a substituted aryl group, an aryl(C₁-C₈)alkyl group and the like; Y represents hydrogen atom or -C(R²)R³ (R² and R³ represent hydrogen atom, a C₁-C₈ alkyl group, a C₁-C₈ alkoxy(C₁-C₈)alkyl group, a hydroxy(C₁-C₈)alkyl group and the like); symbol "a" represents single bond when Y represents hydrogen atom, or "a" represents double bound when Y represents -C(R²)R³; -A-B- represents -CH₂-CH₂-, -S-CH₂-, -O-CH₂-, -CH₂-S-, -CH₂-O-, -SO-CH₂-, -CH₂-SO-, -SO₂-CH₂-, or -CH₂-SO₂-; and Z represents -CH₂- or single bond.

## Description

### Technical Field

The present invention relates to a poly(ADP-ribose)polymerase inhibitor comprising a pyrimidine derivative, and a medicament for therapeutic treatment of a brain disease which comprises a pyrimidine derivative.

### Background Art

Poly(ADP-ribose)polymerase (hereinafter abbreviated as "PARP") is an intranuclear protein that regulates DNA functions, which has been focused recently as an apoptosis relating enzyme. The enzyme, which is activated after recognition of a nick of DNA damaged by nitrogen monoxide and the like or radical oxygen species such as active oxygen generated in foci under ischemia, acts to sequentially bind poly(ADP-ribose) to an acceptor protein using NAD (nicotinamide adenine dinucleotide) as an enzyme substrate which is an intracellular essential constituting element. It is believed that uncontrolled hyperactivation of this enzyme, which is considered to be positioned at the bottom level of the signal transfer system of radical-induced cellular damage, induces decrease of intracellular energy producibility due to NAD exhaustion and oxidative environment in the cells, and finally leads to cell death.

It has been recently reported that, in an experiment using a PARP-knockout mouse whose PARP gene is artificially deleted, cultured nerve cells collected from the brain of the knockout mouse have resistance to damages caused by nitrogen monoxide or an excitatory amino acid such as NMDA (N-methyl-D-aspartate), and moreover, the knockout mouse exhibited a surprisingly high protective effect, i.e., about 80% or more suppression of cerebral infarction foci caused by cerebral ischemia (Eliasson MJL. et al., Nature Med., 3, 1089-95(1997)). Accordingly, it has strongly been suggested that PARP inhibitors can be applied to nerve degenerative diseases such as stroke, Alzheimer's disease, Huntington's chorea, and Parkinson's disease. As for other diseases, a report indicates that the inhibitors are also effective for circulatory diseases such as myocardial infarction, diabetes and septic shock, and inflammatory diseases such as chronic articular rheumatism and multiple sclerosis (Szabo C. et al., Trend Pharmacol. Sci., 19, 287-98(1998)).

Most of PARP inhibitors reported so far have a benzamide structure or a nicotinamide structure in their molecules (JP64-9980, EP0355750, GB2244646, GB2281860, WO94/09776, WO95/20952, WO95/24379, WO96/28167, US5589483, WO97/04771). However, inhibitors with low toxicity which meet the aforementioned purpose have not yet been found.

Among the compounds of formula (I) shown below, 3,5,7,8-tetrahydro-thiopyrano[4,3-d]pyrimidin-4-one derivatives wherein R is hydrogen atom, a C₁-C₈ alkyl group, an aryl group, a methyl-substituted aryl group, an aryl(C₁-C₈)alkyl group, hydroxyl, mercapto, a C₁-C₈ alkylmercapto or amino group; Y is hydrogen atom, "a" is single bond, -A-B- is -S-CH₂-, and Z is -CH₂- are described in US Patent Nos. 2,635,101 and 3,316,257. However, these compounds are intended to be used as a medicament for circulatory system (e.g., a medicament having dilatory action on the coronary artery or diuretic action). In addition, there is no suggestion about PARP inhibitory activity of these compounds in the aforementioned publications.

### Disclosure of the Invention

In view of the circumstances, an object of the present invention is to provide a medicament for therapeutic treatment of diseases in which PARP is involved by finding a low-toxic and potent PARP inhibitor having an apparently different structure from those of the PARP inhibitors having been reported so far.

The inventors of the present invention conducted intensive studies and found that the pyrimidine derivative represented by the following formula (I) strongly inhibited PARP. The present invention was achieved on the basis of the findings.

The present invention provides a medicament for therapeutic and/or preventive treatment of a brain disease, which comprises a compound represented by the following general formula (I) or a pharmaceutically acceptable salt thereof as an active ingredient: 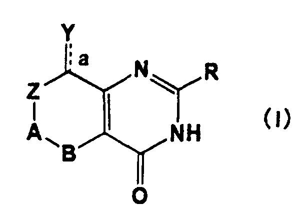 wherein R represents hydrogen atom, a C₁-C₈ alkyl group, a substituted C₁-C₈ alkyl group, an aryl group, a substituted aryl group, an aryl(C₁-C₈)alkyl group, a substituted aryl(C₁-C₈)alkyl group, hydroxyl group, mercapto group, a C₁-C₈ alkylmercapto group, amino group, a C₁-C₈ alkylamino group, a dialkylamino group, NHCOR¹ (R¹ represents hydrogen atom, a C₁-C₈ alkyl group, an aryl group, a substituted aryl group, an aryl(C₁-C₈)alkyl group, or a substituted aryl(C₁-C₈)alkyl group), a C₁-C₈ alkoxyl group, phenoxy group, a substituted phenoxy group, a heterocyclic group, or a substituted heterocyclic group;
Y represents hydrogen atom or -C(R²)R³ (R² and R³ are the same or different and represent hydrogen atom, a C₁-C₈ alkyl group, a C₁-C₈ alkoxy(C₁-C₈)alkyl group, a hydroxy(C₁-C₈)alkyl group, an aryl(C₁-C₈)alkyl group, an aryl group, or a heterocyclic group);
symbol "a" represents single bond when Y represents hydrogen atom, or represents double bond when Y represents -C(R²)R³;
-A-B- represents -CH₂-CH₂-, -S-CH₂-, -O-CH₂-, -CH₂-S-, -CH₂-O-, -SO-CH₂-, -CH₂-SO-, -SO₂-CH₂-, or -CH₂-SO₂-; and
Z represents -CH₂- or single bond; a medicament having anti-inflammatory action, which comprises the compound represented by the aforementioned general formula (I) or a pharmaceutically acceptable salt thereof as an active ingredient; and a use of the compound represented by the aforementioned general formula (I) or a pharmaceutically acceptable salt thereof for manufacture of the aforementioned medicaments. The present invention also provides a poly(ADP-ribose)polymerase inhibitor which comprises the compound represented by the aforementioned general formula (I) or a pharmaceutically acceptable salt thereof.

From another aspect, there are provided a method for therapeutic and/or preventive treatment of a brain disease, which comprises the step of administering a therapeutically and/or preventively effective amount of the compound represented by the aforementioned general formula (I) or a pharmaceutically acceptable salt thereof to a mammal including a human; and a method for therapeutic and/or preventive treatment of an inflammatory disease, which comprises the step of administering a therapeutically and/or preventively effective amount of the compound represented by the aforementioned general formula (I) or a pharmaceutically acceptable salt thereof to a mammal including a human.

From further aspect, the present invention provides a novel compound which falls within the scope of the compound represented by the aforementioned general formula (I).

### Best Mode for Carrying out the Invention

### Definitions

In descriptions of the present specification relating to chemical substances and manufacture thereof, an alkyl group or an alkyl group that constitutes a part of a substituted group means an alkyl group having from 1 to 8 carbon atoms unless otherwise specifically mentioned, and includes a straight alkyl group represented typically by methyl group, ethyl group and the like; a branched alkyl group such as isopropyl group, isobutyl group, and t-butyl group; a cyclic alkyl group such as cyclopropyl group, cyclobutyl group, and cyclohexyl group; and an alkyl group which is a combination of a straight or branched alkyl group and a cyclic alkyl group such as cyclopropylmethyl group, cyclopropylethyl group, and cyclobutylmethyl group.

"Lower" means that the substituent has from 1 to 6 carbon atoms.

A halogen atom means an atom of fluorine, chlorine, bromine or iodine.

An aryl group, or an aryl group that constitutes a part of a substituted group means a 6 to 14 membered (from monocyclic to tricyclic, preferably from monocyclic to bicyclic) aromatic ring such as phenyl, 1-naphthyl, 2-naphthyl, biphenyl, and 2-anthrylnaphthyl.

A heteroring means a 5 to 14 membered, preferably 5 to 10 membered, (from monocyclic to tricyclic, preferably monocyclic or bicyclic) heteroring which contains 1 to 4 heteroatoms of one or two kinds selected from nitrogen atom, oxygen atom and sulfur atom other than carbon atom. Examples include, for example, thiophene, pyridine, furan, pyrazole, pyrazine and the like. A heterocyclic group means a residue of the aforementioned heteroring.

### Compound

In the formula (I), the alkyl group represented by R, R¹, R², or R³, or an alkyl group existing on the substituent represented by R, R¹, R² or R³ is preferably a C₁-C₈ alkyl group, more preferably a C₁-C₆ alkyl group, further preferably a C₁-C₄ alkyl group. When the alkyl group represented by R, R¹, R², or R³ is a cyclic alkyl group, the alkyl group is preferably a C₃-C₇ cycloalkyl group, which includes, for example, cyclopropyl, cyclohexyl and the like.

One or more hydrogen atoms on the alkyl group represented by R may be substituted. Examples of such substituent include a halogen atom, nitro group, cyano group, hydroxyl group, a lower alkoxyl group (e.g., a C₁₋₆ alkoxyl group such as methoxy, ethoxy, propoxy, isopropoxy, butoxy, isobutoxy, pentyloxy, hexyloxy and the like), amino group, a mono(lower alkyl)amino group (e.g., a mono(C₁₋₆ alkyl)amino group such as methylamino group, ethylamino group and the like), a di(lower alkyl)amino group (e.g., a di(C₁₋₆ alkyl)amino group such as dimethylamino group, diethylamino group and the like), carboxyl group, a lower alkylcarbonyl group (e.g., a (C₁₋₆ alkyl)carbonyl group such as acetyl group, propionyl group and the like), a lower alkoxycarbonyl group (e.g., a (C₁₋₆ alkoxy)carbonyl group such as methoxycarbonyl group, ethoxycarbonyl group, propoxycarbonyl group, butoxycarbonyl group and the like), carbamoyl group, a mono(lower alkyl)carbamoyl group (e.g., a mono(C₁₋₆ alkyl)carbamoyl group such as methylcarbamoyl group, ethylcarbamoyl group and the like), a di(lower alkyl)carbamoyl group (e.g., a di(C₁₋₆ alkyl)carbamoyl group such as dimethylcarbamoyl group, diethylcarbamoyl group and the like), an arylcarbamoyl group (e.g., a (C₆₋₁₀ aryl)carbamoyl group such as phenylcarbamoyl group, naphthylcarbamoyl group and the like), an aryloxy group (e.g., a C₆₋₁₀ aryloxy group such as phenyloxy group, naphthyloxy group and the like), an optionally halogenated lower alkylcarbonylamino group (e.g., an optionally halogenated (C₁₋₆ alkyl)carbonylamino group such as acetylamino group, trifluoroacetylamino group and the like), oxo group, a halogen-substituted or an alkyl-substituted aryloxy group, an arylsulfanyl group, a halogen-substituted arylsulfanyl group, a heterocyclic group and the like. Preferred substituents include a halogen atom, hydroxyl group, a lower alkoxyl group, an aryloxy group (e.g., a C₆₋₁₀ aryloxy group such as phenyloxy group and naphthyloxy group), a halogen-substituted or an alkyl-substituted aryloxy group, an arylsulfanyl group, a halogen-substituted arylsulfanyl group, and a heterocyclic group. When two or more substituents exist on the alkyl group, they may be the same or different.

One or more hydrogen atoms of the aryl group represented by R, R¹, R² or R³, or those of an aryl group existing on the substituent represented by R, R¹, R² or R³ may be substituted. Examples of the substituent include a halogen atom, nitro group, cyano group, hydroxyl group, an optionally halogenated lower alkyl group (e.g., an optionally halogenated C₁₋₆ alkyl group such as methyl group, chloromethyl group, difluoromethyl group, trichloromethyl group, trifluoromethyl group, ethyl group, 2-bromoethyl group, 2,2,2-trifluoroethyl group, pentafluoroethyl group, propyl group, 3,3,3-trifluoropropyl group, isopropyl group, butyl group, isobutyl group, sec-butyl group, tert-butyl group, 4,4,4-trifluorobutyl group, pentyl group, isopentyl group, neopentyl group, 5,5,5-trifluoropentyl group, hexyl group, 6,6,6-trifluorohexyl group and the like), a lower alkoxyl group (e.g., a C₁₋₆ alkoxyl group such as methoxy group, ethoxy group, propoxy group, isopropoxy group, butoxy group, isobutoxy group, pentyloxy group, hexyloxy group and the like), amino group, a mono(lower alkyl)amino group (e.g., a mono(C₁₋₆ alkyl)amino group such as methylamino group and ethylamino group and the like), a di(lower alkyl)amino group (e.g., a di(C₁₋₆ alkyl)amino group such as dimethylamino group, diethylamino group and the like), carboxyl group, a lower alkylcarbonyl group (e.g., a (C₁₋₆ alkyl)carbonyl group such as acetyl group, propionyl group and the like), a lower alkoxycarbonyl group (e.g., a (C₁₋₆ alkoxy)carbonyl group such as methoxycarbonyl group, ethoxycarbonyl group, propoxycarbonyl group, butoxycarbonyl group and the like), carbamoyl group, a mono(lower alkyl)carbamoyl group (e.g., a mono(C₁₋₆ alkyl)carbamoyl group such as methylcarbamoyl group, ethylcarbamoyl group and the like), a di(lower alkyl)carbamoyl group (e.g., a di(C₁₋₆ alkyl)carbamoyl group such as dimethylcarbamoyl group, diethylcarbamoyl group and the like), an arylcarbamoyl group (e.g., a (C₆₋₁₀ aryl)carbamoyl group such as phenylcarbamoyl group, naphthylcarbamoyl group and the like), an aryl group (e.g., a C₆₋₁₀ aryl group such as phenyl group, naphthyl group and the like), an aryloxy group (e.g., a C₆₋₁₀ aryloxy group such as phenyloxy group, naphthyloxy group and the like), an optionally halogenated lower alkylcarbonylamino group (e.g., an optionally halogenated (C₁₋₆ alkyl)carbonylamino group such as acetylamino group, trifluoroacetylamino group and the like), oxo group and the like; preferably a halogen atom, nitro group, and an optionally halogenated lower alkyl group.

One or more hydrogen atoms on the phenoxy group represented by R¹ may be substituted. Examples of the substituent include a halogen atom, nitro group, cyano group, hydroxyl group, an optionally halogenated lower alkyl group (e.g., an optionally halogenated C₁₋₆ alkyl group such as methyl group, chloromethyl group, difluoromethyl group, trichloromethyl group, trifluoromethyl group, ethyl group, 2-bromoethyl group, 2,2,2-trifluoroethyl group, pentafluoroethyl group, propyl group, 3,3,3-trifluoropropyl group, isopropyl group, butyl group, isobutyl group, sec-butyl group, tert-butyl group, 4,4,4-trifluorobutyl group, pentyl group, isopentyl group, neopentyl group, 5,5,5-trifluoropentyl group, hexyl group, 6,6,6-trifluorohexyl group and the like), a lower alkoxyl group (e.g., a C₁₋₆ alkoxyl group such as methoxy group, ethoxy group, propoxy group, isopropoxy group, butoxy group, isobutoxy group, pentyloxy group, hexyloxy group and the like), amino group, a mono(lower alkyl)amino group (e.g., a mono(C₁₋₆ alkyl)amino group such as methylamino group, ethylamino group and the like), a di(lower alkyl)amino group (e.g., a di(C₁₋₆ alkyl)amino group such as dimethylamino group, diethylamino group and the like), carboxyl group, a (lower alkyl)carbonyl group (e.g., a (C₁₋₆ alkyl)carbonyl group such as acetyl group, propionyl group and the like), a (lower alkoxyl)carbonyl group (e.g., a (C₁₋₆ alkoxyl)carbonyl group such as methoxycarbonyl group, ethoxycarbonyl group, propoxycarbonyl group, butoxycarbonyl group and the like), carbamoyl group, a mono(lower alkyl)carbamoyl group (e.g., a mono(C₁₋₆ alkyl)carbamoyl group such as methylcarbamoyl group, ethylcarbamoyl group and the like), a di(lower alkyl)carbamoyl group (e.g., a di(C₁₋₆ alkyl)carbamoyl group such as dimethylcarbamoyl group, diethylcarbamoyl group and the like), an arylcarbamoyl group (e.g., a (C₆₋₁₀ aryl)carbamoyl group such as phenylcarbamoyl group and naphthylcarbamoyl group), an aryl group (e.g., a C₆₋₁₀ aryl group such as phenyl group, naphthyl group and the like), an aryloxy group (e.g., a C₆₋₁₀ aryloxy group such as phenyloxy group, naphthyloxy group and the like), an optionally halogenated lower alkylcarbonylamino group (e.g., an optionally halogenated (C₁₋₆ alkyl)carbonylamino group such as acetylamino group, trifluoroacetylamino group and the like), oxo group and the like; preferably a halogen atom, and a lower alkyl group.

One or more hydrogen atoms on the heterocyclic group represented by R, R², or R³ may be substituted. Examples of the substituent include a C₁₋₆ alkyl group and a halogen-substituted C₁₋₆ alkyl group.

In the formula (I), the group represented by R is preferably hydrogen atom, a C₁-C₈ alkyl group (such as methyl group, ethyl group, isopropyl group, tert-butyl group, n-hexyl group and the like), a substituted C₁-C₈ alkyl group [such as a monofluoroalkyl group, a monochloroalkyl group, a hydroxyalkyl group, phenoxymethyl group, a substituted phenoxymethyl group (the substituent on the phenyl group includes a lower alkyl group, fluorine atom, chlorine atom and the like), phenylthiomethyl group, a substituted phenylthiomethyl group (the substituent on the phenyl group includes chlorine atom and the like) and the like], an aryl group (such as phenyl group and the like), a substituted aryl group (the substituent on the aryl group includes nitro group, trifluoromethyl group and the like), an aryl(C₁-C₈)alkyl group (such as benzyl group, phenethyl group and the like), a substituted aryl(C₁-C₈)alkyl group (the substituent on the aryl group includes fluorine atom and the like), a C₁-C₈ alkoxyl group (such as methoxy group and the like), a heterocyclic group (such as pyridyl group, pyridazinyl group, thienyl group, pyrazolyl group and the like), or a substituted heterocyclic group (the substituent on the heteroring includes trifluoromethyl group and the like); further preferably a C₁-C₈ alkyl group, a substituted C₁-C₈ alkyl group, an aryl group, a substituted aryl group, and an aryl(C₁-C₈)alkyl group.

In the formula (I), the group represented by Y is preferably hydrogen atom or -C(R²)R³ [R² and R³ are the same or different and represent hydrogen atom, a C₁-C₈ alkyl group (such as methyl group, ethyl group, isopropyl group, cyclohexyl group and the like), a C₁-C₈ alkoxy(C₁-C₈)alkyl group (such as methoxymethyl group, methoxyethyl group and the like), a hydroxy(C₁-C₈)alkyl group (such as hydroxymethyl group and the like), an aryl(C₁-C₈)alkyl group (such as benzyl group, phenethyl group and the like), an aryl group (such as phenyl group and the like), a heterocyclic group (such as thienyl group , furyl group and the like)]; preferably hydrogen atom or -C(R²)R³ (both R² and R³ are hydrogen atoms, or R² is hydrogen atom and R³ represents the aforementioned substituent); and further preferably, Y is hydrogen atom.

In the formula (I), -A-B- is preferably -CH₂-CH₂-, -S-CH₂-, -O-CH₂-, -CH₂-S-, -SO-CH₂-, or -SO₂-CH₂-, further preferably -S-CH₂-.

In the formula (I), Z is preferably -CH₂-.

A class of preferred compounds among the compounds represented by the formula (I) are those wherein R is hydrogen atom, a C₁-C₈ alkyl group, a substituted C₁-C₈ alkyl group, an aryl group, a substituted aryl group, an arylalkyl group, a substituted arylalkyl group, a C₁-C₈ alkoxyl group, a heterocyclic group, or a substituted heterocyclic group;
Y is hydrogen atom or -C(R²)R³ (R² and R³ are the same or different and represent hydrogen atom, a C₁-C₈ alkyl group, a C₁-C₈ alkoxy(C₁-C₈)alkyl group, a hydroxy(C₁-C₈)alkyl group, an aryl(C₁-C₈)alkyl group, an aryl group, or a heterocyclic group);
when Y represents hydrogen atom, symbol "a" is single bond; or when Y represents -C(R²)R³, symbol "a" is double bond;
-A-B- is -CH₂-CH₂-, -S-CH₂-, -O-CH₂-, -CH₂-S-, -SO-CH₂-, or -SO₂-CH₂-; and
Z is -CH₂- or a bond (specific examples of each substituent are those explained above).

Further preferred compounds include those wherein:
R is a C₁-C₈ alkyl group, a substituted C₁-C₈ alkyl group, an aryl group, a substituted aryl group, or an arylalkyl group;
Y is hydrogen atom;
symbol "a" is single bond;
-A-B- is -S-CH₂-; and
Z is -CH₂-.

Preferred specific examples of the compound of the formula (I) include:
2-(4-trifluoromethylphenyl)-3,5,7,8-tetrahydrothiopyrano[4,3-d]pyrimidin-4-one,
2-methyl-3,5,7,8-tetrahydrothiopyrano[4,3-d]pyrimidin-4-one,
3,5,7,8-tetrahydrothiopyrano[4,3-d]pyrimidin-4-one,
2-phenyl-3,5,7,8-tetrahydrothiopyrano[4,3-d]pyrimidin-4-one,
2-methoxy-3,5,7,8-tetrahydrothiopyrano[4,3-d]pyrimidin-4-one,
2-methyl-5,6,7,8-tetrahydro-3H-quinazolin-4-one,
2-phenyl-5,6,7,8-tetrahydro-3H-quinazolin-4-one,
2-phenyl-7,8-tetrahydro-3H,6H-thiopyrano[3,2-d]pyrimidin-4-one,
2-phenyl-3,5,7,8-tetrahydropyrano[4,3-d]pyrimidin-4-one,
2-methyl-3,5,7,8-tetrahydropyrano[4,3-d]pyrimidin-4-one,
2-benzyl-3,5,7,8-tetrahydrothiopyrano[4,3-d]pyrimidin-4-one,
2-(3-nitrophenyl)-3,5,7,8-tetrahydrothiopyrano[4,3-d]pyrimidin-4-one,
2-(4-fluorobenzyl)-3,5,7,8-tetrahydrothiopyrano[4,3-d]pyrimidin-4-one
2-(3-pyridyl)-3,5,7,8-tetrahydrothiopyrano[4,3-d]pyrimidin-4-one,
2-methyl-6,7-dihydro-3H-thieno[3,2-d]pyrimidin-4-one,
2-phenyl-6,7-dihydro-3H-thieno[3,2-d]pyrimidin-4-one,
2-(3,5-dimethyl-pyrazol-1-yl)-3,5,7,8-tetrahydrothiopyrano[4,3-d]pyrimidin-4-one,
6,7-dihydro-3H-thieno[3,2-d]pyrimidin-4-one,
2-methyl-8-methylene-3,5,7,8-tetrahydrothiopyrano[4,3-d]pyrimidin-4-one,
2-methyl-8-methylene-5,6,7,8-tetrahydro-3H-quinazolin-4-one,
2-phenyl-8-methylene-5,6,7,8-tetrahydro-3H-quinazolin-4-one,
2-phenyl-8-methylene-3,5,7,8-tetrahydrothiopyrano[4,3-d]pyrimidin-4-one,
2-thiophen-2-yl-3,5,7,8-tetrahydrothiopyrano[4,3-d]pyrimidin-4-one,
2-(4-chlorophenoxymethyl)-3,5,7,8-tetrahydrothiopyrano[4,3-d]pyrimidin-4-one,
2-hexyl-3,5,7,8-tetrahydrothiopyrano[4,3-d]pyrimidin-4-one,
2-phenoxymethyl-3,5,7,8-tetrahydrothiopyrano[4,3-d]pyrimidin-4-one,
2-(4-fluoro-phenoxymethyl)-3,5,7,8-tetrahydrothiopyrano[4,3-d]pyrimidin-4-one,
2-(3-fluorophenoxymethyl)-3,5,7,8-tetrahydrothiopyrano[4,3-d]pyrimidin-4-one,
2-(2-fluoro-phenoxymethyl)-3,5,7,8-tetrahydrothiopyrano[4,3-d]pyrimidin-4-one,
2-(3-hydroxypropyl)-3,5,7,8-tetrahydrothiopyrano[4,3-d]pyrimidin-4-one,
2-methyl-8-propylidene-3,5,7,8-tetrahydrothiopyrano[4,3-d]pyrimidin-4-one,
8-ethylidene-2-methyl-3,5,7,8-tetrahydrothiopyrano[4,3-d]pyrimidin-4-one,
2-methyl-8-(2-methylpropylidene)-3,5,7,8-tetrahydrothiopyrano[4,3-d]-pyrimidin-4-one,
8-cyclohexylmethylene-2-methyl-3,5,7,8-tetrahydrothiopyrano[4,3-d]-pyrimidin-4-one,
2-(3-hydroxypentyl)-3,5,7,8-tetrahydrothiopyrano[4,3-d]pyrimidin-4-one,
8-(2-methoxyethylidene)-2-methyl-3,5,7,8-tetrahydrothiopyrano[4,3-d]-pyrimidin-4-one,
8-(2-methoxypropylidene)-2-methyl-3,5,7,8-tetrahydrothiopyrano[4,3-d]-pyrimidin-4-one,
8-(2-hydroxyethylidene)-2-methyl-3,5,7,8-tetrahydrothiopyrano[4,3-d]-pyrimidin-4-one,
2-(2-chlorophenoxymethyl)-3,5,7,8-tetrahydrothiopyrano[4,3-d]pyrimidin-4-one,
2-(2-tert-butyl)-3,5,7,8-tetrahydrothiopyrano[4,3-d]pyrimidin-4-one,
2-(2-pyrazin-2-yl)-3,5,7,8-tetrahydrothiopyrano[4,3-d]pyrimidin-4-one,
2-(5-trifluoromethylpyridin-2-yl)-3,5,7,8-tetrahydrothiopyrano[4,3-d]-pyrimidin-4-one,
2-(4-chlorophenyl)-3,5,7,8-tetrahydrothiopyrano[4,3-d]pyrimidin-4-one,
2-(2-isopropyl)-3,5,7,8-tetrahydrothiopyrano[4,3-d]pyrimidin-4-one,
2-methyl-3,5,7,8-tetrahydrothiopyrano[4,3-d]pyrimidin-4-one-6-oxide,
2-methyl-3,5,7,8-tetrahydrothiopyrano[4,3-d]pyrimidin-4-one-6,6-dioxide,
2-phenylsulfanylmethyl-3,5,7,8-tetrahydrothiopyrano[4,3-d]pyrimidin-4-one,
2-(4-chlorophenylsulfanylmethyl)-3,5,7,8-tetrahydrothiopyrano[4,3-d]-pyrimidin-4-one,
2-(pyridin-4-yl)-3,5,7,8-tetrahydrothiopyrano[4,3-d]pyrimidin-4-one,
2-(pyridin-2-yl)-3,5,7,8-tetrahydrothiopyrano[4,3-d]pyrimidin-4-one,
2-(phenethyl)-3,5,7,8-tetrahydrothiopyrano[4,3-d]pyrimidin-4-one,
2-(3-chlorophenoxymethyl)-3,5,7,8-tetrahydrothiopyrano[4,3-d]pyrimidin-4-one,
2-(2-methylphenoxymethyl)-3,5,7,8-tetrahydrothiopyrano[4,3-d]pyrimidin-4-one,
2-(2-bromophenoxymethyl)-3,5,7,8-tetrahydrothiopyrano[4,3-d]pyrimidin-4-one,
2-ethyl-3,5,7,8-tetrahydrothiopyrano(4,3-d]pyrimidin-4-one,
2-fluoromethyl-3,5,7,8-tetrahydrothiopyrano[4,3-d]pyrimidin-4-one,
2-chloromethyl-3,5,7,8-tetrahydrothiopyrano[4,3-d]pyrimidin-4-one,
8-benzylidene-2-methyl-3,5,7,8-tetrahydrothiopyrano[4,3-d]pyrimidin-4-one,
2-methyl-8-(thiophen-3-ylmethylene)-3,5,7,8-tetrahydrothiopyrano[4,3-d]-pyrimidin-4-one,
8-(furan-2-ylmethylene)-2-methyl-3,5,7,8-tetrahydrothiopyrano[4,3-d]-pyrimidin-4-one,
2-methyl-8-phenethylidene-3,5,7,8-tetrahydrothiopyrano[4,3-d]pyrimidin-4-one,
2-methyl-8-(3-phenylpropylidene)-3,5,7,8-tetrahydrothiopyrano[4,3-d]-pyrimidin-4-one, and
2-methyl-7,8-dihydro-3H,6H-thiopyrano[3,2-d]pyrimidin-4-one; however, the scope of the present invention is not limited thereto.

### Preparation of the compound of the formula (I)

Among the compounds of the formula (I) according to the present invention, the compounds wherein Y is hydrogen atom and symbol "a" is single bond can be prepared by a method as shown below.

The compound can be obtained by reacting a compound represented by the following formula (II): 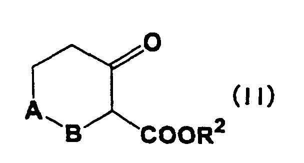 wherein -A-B- has the same meaning as that defined in the formula (I) and R⁵ represents a C₁-C₈ alkyl group (preferably methyl group, ethyl group), benzyl group and the like, with a compound represented by the following formula (III): 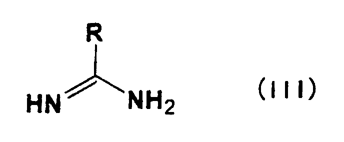 wherein R has the same meaning as that defined in the formula (I), in a solvent which does not participate in the reaction (e.g., water, an alcohol, tetrahydrofuran, dioxane, dimethylformamide) in the presence of an alkali carbonate such as potassium carbonate or an organic amine such as triethylamine at a reaction temperature from 20 to 120°C, preferably from 40 to 80°C, for from 1 to 24 hours, usually from 2 to 15 hours.

Among the compounds of the formula (I) according to the present invention, the compounds wherein Y is -C(R²)R³ (R² and R³ are the same or different and represent hydrogen atom, a C₁-C₈ alkyl group, a C₁-C₈ alkoxy(C₁-C₈)alkyl group, a hydroxy(C₁-C₈)alkyl group, an aryl(C₁-C₈)alkyl group, an aryl group, or a heteroring) and symbol "a" is double bond can be prepared according to the following steps.

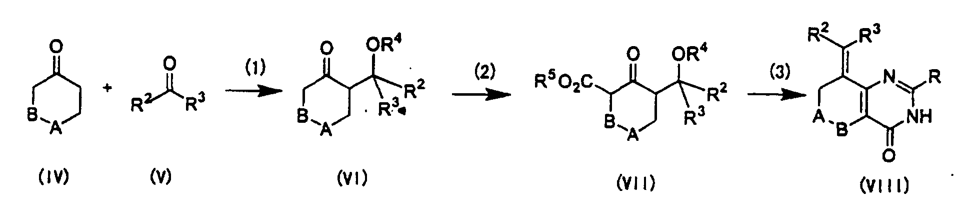

### <Step 1>

A compound of formula (VI) wherein R² and R³ have the same meanings as those defined in the formula (I) and R⁴ represents a protecting group for hydroxyl group can be prepared by treating a ketone derivative represented by formula (IV) wherein -A-B- has the same meaning as that defined in the formula (I) with a base, then condensing the resulting product with a carbonyl derivative represented by formula (V) wherein R² and R³ have the same meanings as those defined in the formula (I) in a reaction solvent such as tetrahydrofuran and diethyl ether (preferably tetrahydrofuran) at a temperature from -78°C to 0°C (preferably from -78°C to -40°C) by using a base, and protecting the resulting hydroxyl group with an appropriate protective group.

In this condensation, an alkyllithium (such as methyllithium, ethyllithium, n-propyllithium, n-butyllithium, sec-butyllithium and t-butyllithium), lithium diisopropylamide and the like (preferably n-butyllithium, lithium diisopropylamide) is used as the base. Alternatively, the compound represented by the formula (IV) may be converted to a trimethylsilyl ether by using a silylating agent such as a trimethylsilyl halide, and then the resulting product may be condensed with a variety of aldehyde or ketone represented by the formula (V) by using a Lewis acid (such as titanium tetrachloride, aluminum chloride, zinc chloride and tin tetrachloride).

In the formula (VI), as the protective group for the hydroxyl group represented by R⁴, various protective groups for hydroxyl group described in Protective Groups in Organic Synthesis (Green, Wuts) may be used. Examples include benzyl group, tert-butyldiphenylsilyl group, tetrahydropyranyl group, ethoxyethyl group and the like, and preferred examples include tetrahydropyranyl group and ethoxyethyl group.

### <Step 2>

A compound of formula (VII) wherein -A-B-, R², R³ and R⁴ have the same meanings as those defined in the formula (VI) and R⁵ represents a C₁-C₈ alkyl group, benzyl group and the like, preferably methyl, ethyl group, can be prepared by treating the compound of the formula (VI) with a base, and then reacting the resulting product with, for example, NCCO₂R⁵ (an alkyl cyanoacetate; R⁵ represents a C₁-C₈ alkyl group, benzyl group and the like, preferably methyl, ethyl group) as a reaction reagent in a reaction solvent such as tetrahydrofuran and diethyl ether (preferably tetrahydrofuran) at a temperature from -78°C to 0°C (preferably from -20°C to 0°C).

As the reaction reagent, an alkyl chloroacetate, an alkyl bromoacetate, an alkyl iodoacetate and the like may also be used in addition to the alkyl cyanoacetate.

Examples of the base include methyllithium, n-butyllithium, lithium diisopropylamide and the like, and a preferred example includes lithium diisopropylamide.

### <Step 3>

A compound represented by formula (VIII) can be obtained by reacting the compound of the formula (VII) with the compound of the formula (III) wherein R has the same meaning as that defined in the formula (I) in a solvent which does not participate in the reaction (e.g., water, an alcohol, tetrahydrofuran, dioxane, dimethylformamide) in the presence of an alkali carbonate such as potassium carbonate or an organic amine such as triethylamine at a reaction temperature from 20 to 120°C, preferably from 20 to 80°C, for 1 to 24 hours, preferably for 2 to 15 hours, and then subjecting the resulting product to a ring formation, deprotection of hydroxyl group, and then dehydration.

In this reaction, when an acetal protective group such as tetrahydropyranyl group and ethoxyethyl group is selected as R⁴, deprotection and dehydration can be carried out simultaneously, thereby the steps can be shortened.

Among the compounds of formula (I) according to the present invention, the compound wherein Y is -C(R²)R³ (both R² and R³ represent hydrogen atom) and symbol "a" is double bond can be prepared according to the following steps.

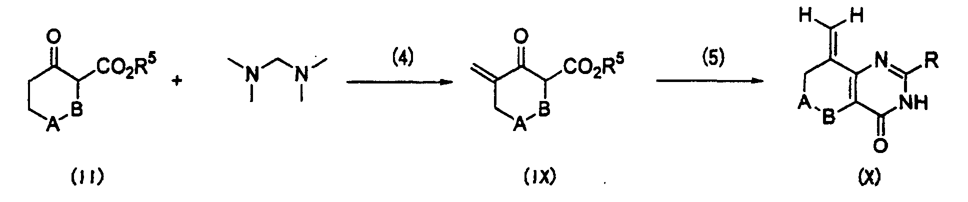

### <Step 4>

The exomethylene derivative represented by formula (IX) wherein -A-B- and R⁵ have the same meanings as those defined in the formula (II) can be synthesized by dissolving the ketone derivative of the formula (II) in acetic anhydride, then adding N,N-tetramethyldiaminomethane to the solution, and carrying out reaction at a temperature from 20°C to 120°C (preferably from 40°C to 80°C) to introduce methylene group.

However, this reaction is not limited to the method described above. For example, a Mannich base, a Eschenmoser reagent (H₂N=(CH₃)₂I), a Brederick reagent ([(CH₃)₂N]₂CHO-t-C₄H₉) and the like may be used as a reagent for introducing the methylene group. As the reaction solvent, dimethylformamide, tetrahydrofuran, diethyl ether and the like may be used.

### <Step 5>

A compound represented by formula (X) can be obtained by reacting the compound represented by the formula (IX) wherein -A-B- and R⁵ have the same meanings as those defined in the formula (II) with the compound represented by the formula (III) wherein R has the same meaning as that defined in the formula (I) in a solvent which does not participate in the reaction (e.g., water, an alcohol, tetrahydrofuran, dioxane, dimethylformamide) in the presence of an alkali carbonate such as potassium carbonate or an organic amine such as triethylamine at a reaction temperature from 20 to 120°C, preferably from 20 to 80°C, for 1 to 24 hours, preferably from 2 to 15 hours to form a ring.

The compound of the aforementioned formula (I) forms a salt with a variety of inorganic and organic acids, and this property is utilized for manufacture of a pure substance or as a form of a medicament provided. More specifically, for the aforementioned manufacture, acidification may convert the compound soluble in a polar solvent such as water and enable extraction or purification to allow isolation of the compound in a form of a salt having preferred physicochemical properties. For use as medicaments, the compound may be in the form a pharmaceutically acceptable salt. In addition, the compound represented by the aforementioned formula (I) may sometimes exist as a hydrate or a solvate other than the free form or the form of a salt thereof. As an active ingredient of the medicament of the present invention, a substance in any form mentioned above may be used.

Examples of the form of a possible salt include an acid addition salt with an inorganic acid such as hydrochloric acid, nitric acid, hydrobromic acid and sulfuric acid, and a salt derived from a nontoxic organic acid such as an aliphatic monocarboxylic acid, a dicarboxylic acid, a hydroxyalkanoic acid, a hydroxyalkanoic diacid, an amino acid, an aromatic acid, an aliphatic or aromatic sulfonic acid and the like. Examples of such acid addition salt include hydrochloride, hydrobromide, nitrate, sulfate, hydrogensulfate, monohydrogenphosphate, dihydrogenphosphate, acetate, propionate, tartrate, oxalate, malonate, succinate, fumarate, maleate, mandelate, benzoate, phthalate, methanesulfonate, benzenesulfonate, toluenesulfonate, citrate, lactate, malate, glycolate and the like.

The acid addition salt mentioned above has a significance from one aspect as a pharmacologically acceptable pharmaceutical composition. The pharmaceutical composition is considered to have an advantage from a viewpoint of manufacturing process for pharmaceuticals and a usefulness from viewpoints of distribution, absorbability and the like when administered to a human.

The compound represented by the aforementioned formula (I) may optionally have one or more asymmetric carbon atoms and exist as a stereoisomer (an optical isomer or a diastereoisomer) based on the asymmetric carbon atom(s). Any mixtures of the stereoisomers or racemates as well as the stereoisomers in a pure form may be used as an active ingredient of the medicament of the present invention. When the compound represented by the aforementioned formula (I) has an olefinic double bond, the compound may exist as a geometrical isomer in either Z- or E-form or a mixture thereof. The geometrical isomer in a pure form or a mixture thereof may be used as an active ingredient of the medicament of the present invention.

The compound of the aforementioned general formula (I) has potent inhibitory activity against PARP. The compound has low toxicity and few side effects, and accordingly, said compound is useful as an active ingredient of a medicament. The compound represented by the general formula (I) acts as a PARP inhibitor in a mammal (e.g., a mouse, rat, hamster, rabbit, cat, dog, bovine, ovine, monkey, human and the like) and is effective for preventive and/or therapeutic treatment of a disease in which PARP is involved, for example, stroke, myocardial infarction, diabetes, septic shock, Alzheimer's disease, Huntington's chorea, Parkinson's disease and the like.

The compound represented by the aforementioned general formula (I) is used as a medicament for a brain disease, especially a medicament for therapeutic and/or preventive treatment of acute phase stroke, Alzheimer's disease, Parkinson's disease, Huntington's chorea and the like, and also as an anti-inflammatory agent.

The pharmaceutical composition which comprises the compound of the formula (I) according to the present invention as an active ingredient can be administered to a human as well as to an animal other than human by either oral or parenteral (e.g., intravenous, intramuscular, subcutaneous, rectal, transdermal) route of administration. Accordingly, the pharmaceutical composition comprising the compound according to the present invention as an active ingredient can be formulated in a form suitable for a route of administration.

Specifically, examples of preparations for oral administration include tablets, capsules, powders, granules, syrups and the like, and examples of preparations for parenteral administration include injections for intravenous or intramuscular administration and the like, rectal preparations, oleaginous suppositories, aqueous suppositories and the like.

Each of these preparations can be manufactured by a conventional method using a pharmaceutical additive such as commonly used excipients, disintegrators, binders, lubricants, and colorants.

Examples of the excipient include lactose, glucose, corn starch, sorbitol, crystalline cellulose and the like; examples of the disintegrator include starch, sodium alginate, gelatin powder, calcium carbonate, calcium citrate, dextrin and the like; examples of the binder include dimethylcellulose, polyvinyl alcohol, polyvinyl ether, methylcellulose, ethyl cellulose, gum arabic, gelatin, hydroxypropyl cellulose, polyvinylpyrrolidone and the like; and examples of the lubricants include talc, magnesium stearate, polyethylene glycol, hardened vegetable oils and the like. The aforementioned injection can be manufactured, if necessary, by adding buffers, pH adjusters, stabilizers and the like.

An amount of the compound of the present invention in the pharmaceutical composition may vary depending on a type of formulation. In general, the amount may be from about 0.1% to about 50% by weight, preferably from about 0.1% to about 20% by weight, based on the total weight of the composition. A dose can be appropriately chosen for each clinical case with consideration of the age, body weight, sexuality of a patient, a type of a disease and degree of severity of the disease and the like. The dose is generally from about 0.1 mg to 100 mg, preferably from 0.1 mg to 30 mg, per day for an adult, and administered once a day or several times a day as divided portions.

The compound represented by the aforementioned formula (I) is a novel compound except the compound wherein:
R is hydrogen atom, a C₁-C₈ alkyl group, an aryl group, a methyl-substituted aryl group, an aryl(C₁-C₈)alkyl group, hydroxyl group, mercapto group, a C₁-C₈ alkylmercapto group, or amino group;
Y is hydrogen atom;
symbol "a" is single bond;
-A-B- is -CH₂-CH₂-, -S-CH₂-, -CH₂-S-, -SO-CH₂-, or -SO₂-CH₂-; and
Z is -CH₂-.

Preferably, in the aforementioned novel compound, R is hydrogen atom, a C₁-C₈ alkyl group, a substituted C₁-C₈ alkyl group, an aryl group, a substituted aryl group, an arylalkyl group, a substituted arylalkyl group, a C₁-C₈ alkoxyl group, a heterocyclic group, or a substituted heterocyclic group; and -A-B- is -CH₂-CH₂-, -S-CH₂-, -O-CH₂-, -CH₂-S-, -SO-CH₂-, or -SO₂-CH₂-.

The scope of the aforementioned novel compound provided by the present invention encompasses the compound in a free form and a salt thereof, and hydrates thereof and solvates thereof. In addition, the scope of the present invention also encompasses any of stereoisomers based on one or more asymmetric carbon atoms, any mixtures of the stereoisomers, racemates, geometrical isomers based on one or more olefinic double bonds, and any mixtures of the geometrical isomers.

### Examples

The present invention will be further explained in detail by the following examples and other descriptions. However, the present invention is not limited to the examples.

### Preparation example 1: Methyl 4-oxotetrahydrothiopyrane-3-carboxylate

A solution (100 ml) of dimethyl 3,3'-thiodipropionate (8.25 g) in anhydrous tetrahydrofuran was added with sodium hydride (2.11 g) and heated under reflux for 5 hours. The reaction mixture was cooled, then added with acetic acid (8 ml), poured into water, and extracted with ether. The organic layer was dried over anhydrous magnesium sulfate and then evaporated under reduced pressure to obtain the above-titled product.

### Example 1: 2-(4-Trifluoromethylphenyl)-3.5.7.8-tetrahydrothiopyrano[4,3-d]-pyrimidin-4-one

A solution of methyl 4-oxotetrahydrothiopyrane-3-carboxylate (1.86 g) and 4-trifluoromethylbenzamidine (1.92 g) in methanol (15 ml) was added with potassium carbonate (1.41 g), and heated under reflux for 10 hours. The reaction mixture was cooled, then poured into water, and extracted with dichloromethane. The organic layer was dried over anhydrous magnesium sulfate and then evaporated under reduced pressure, and the residue was purified by silica gel column chromatography to obtain the above-titled product (2.24 g).
¹H-NMR (CDCl₃) δ : 2.96 (2H, t, J=5.8Hz), 3.08 (2H, t, J=5.8Hz), 3.72 (2H, s), 7.80 (2H, d, J=8.2Hz), 8.33 (2H, d, J=8.2Hz).
Mass (EI, m/z): 312(M⁺).

### Example 2: 2-Methyl-3,5,7,8-tetrahydrothiopyrano[4,3-d]pyrimidin-4-one

The above-titled product was obtained in the same method as in Example 1 except that acetamidine was used instead of 4-trifluoromethylbenzamidine.
¹H-NMR (CDCl₃) δ : 2.44 (3H, s), 2.87-2.95 (4H, m), 3.61 (2H, s).
Mass (EI, m/z): 182(M⁺).

### Example 3: 3,5,7,8-Tetrahydrothiopyrano[4,3-d]pyrimidin-4-one

The above-titled product was obtained in the same method as in Example 1 except that formamidine was used instead of 4-trifluoromethylbenzamidine.
¹H-NMR (CDCl₃) δ : 2.91 (2H, t, J=5.6Hz), 2.99 (2H, t, J=5.6Hz), 3.64 (2H, s), 8.03 (1H, s).
Mass (EI, m/z): 168(M⁺).

### Example 4: 2-Phenyl-3,5,7,8-tetrahydrothiopyrano[4,3-d]pyrimidin-4-one

The above-titled product was obtained in the same method as in Example 1 except that benzamidine was used instead of 4-trifluoromethylbenzamidine.
¹H-NMR (CDCl₃) δ : 2.94 (2H, t, J=5.6Hz), 3.06(2H, t, J=5.6Hz), 3.71 (2H, s), 7.50-7.60 (3H, m), 8.07-8.11 (2H, m).
Mass (EI, m/z): 244(M⁺).

### Example 5: 2-Methoxy-3,5,7,8-tetrahydrothiopyrano[4,3-d]pyrimidin-4-one

The above-titled product was obtained in the same method as in Example 1 except that 2-methylisourea was used instead of 4-trifluoromethylbenzamidine.
¹H-NMR (CDCl₃) δ : 2.84-2.89 (4H, m), 3.58 (2H, s), 3.96(3H, s).
Mass (EI, m/z): 198(M⁺).

### Example 6: 2-Methyl-5,6,7,8-tetrahydro-3H-quinazolin-4-one

A solution of ethyl 4-oxotetrahydropyrane-3-carboxylate (510 mg) and acetamidine hydrochloride (284 mg) in methanol (5 ml) was added with potassium carbonate (816 mg) and heated under reflux for 5 hours. The reaction mixture was cooled, then poured into water, and extracted with dichloromethane. The organic layer was dried over anhydrous magnesium sulfate and then evaporated under reduced pressure, and the residue was purified by silica gel column chromatography to obtain the above-titled product (374 mg).
¹H-NMR (CDCl₃) δ : 1.71-1.84 (4H, m), 2.42 (3H, s), 2.50(2H, t, J=6.2Hz), 2.61 (2H, t, J=6.2Hz).
Mass (EI, m/z): 164(M⁺).

### Example 7: 2-Phenyl-5,6,7,8-tetrahydro-3H-quinazolin-4-one

The above-titled product was obtained in the same method as in Example 6 except that benzamidine was used instead of acetamidine hydrochloride.
¹H-NMR (CDCl₃) δ : 1.75-1.90 (4H, m), 2.59 (2H, t, J=6.3Hz), 2.74 (2H, t, J=6.3Hz), 7.48-7.55(3H, m), 8.07-8.12(2H, m).
Mass (EI, m/z): 226(M⁺).

### Example 8: 2-Phenyl-7,8-tetrahydro-3H,6H-thiopyrano[3,2-d]pyrimidin-4-one

A solution of ethyl 3-oxo-tetrahydrothiopyrane-2-carboxylate (565 mg), which was synthesized by the method described in Tetrahedron Vol. 37, 2633 (1981), and benzamidine hydrochloride (470 mg) in methanol (5 ml) was added with potassium carbonate (816 mg), and heated under reflux for 10 hours. The reaction mixture was cooled and then poured into water, and the deposited precipitate was collected by filtration and recrystallized from methanol/dichloromethane to obtain the above-titled product (562 mg).
¹H-NMR (CDCl₃) δ : 2.21-2.28 (2H, m), 2.86(2H, t, J=6.3Hz), 3.01-3.05(2H, s), 7.51-7.55 (3H, m), 8.07-8.11(2H, m).
Mass (EI, m/z): 244(M⁺).

### Preparation example 2: Methyl 4-oxotetrahydropyrane-3-carboxvlate

Under argon atmosphere, an anhydrous tetrahydrofuran solution (7.5 ml) of diisopropylamine (0.51 ml) was added with a tetrahydrofuran solution (1.5M, 2.4 ml) of n-butyllithium at -20°C, and stirred for 30 minutes. The reaction mixture was cooled to -78°C and added with tetrahydro-4H-pyran-4-one (300 mg). After the temperature was gradually elevated to 0°C over 1 hour, the mixture was again cooled to -78°C, and added with hexamethylphosphoric triamide (0.3 ml) and methyl cyanoformate (0.29 ml). After the completion of the reaction, the reaction mixture was poured into water and extracted with ether, and the organic layer was dried over anhydrous magnesium sulfate and evaporated under reduced pressure to obtain the above-titled product (120 mg).

### Example 9: 2-Phenyl-3,5,7,8-tetrahydropyrano[4,3-d]pyrimidin-4-one

A solution of methyl 4-oxotetrahydropyrane-3-carboxylate (112 mg) and benzamidine hydrochloride (470 mg) in methanol (2 ml) was added with potassium carbonate (100 mg) and heated under reflux for 3 hours. The reaction mixture was cooled, then poured into water, and extracted with dichloromethane. The organic layer was dried over anhydrous magnesium sulfate and then evaporated under reduced pressure, and the residue was purified by silica gel column chromatography to obtain the above-titled product (90 mg).
¹H-NMR (CDCl₃) δ : 2.83 (2H, t, J=5.6Hz), 4.02(2H, t, J=5.6Hz), 4.67 (2H, s), 7.50-7.60 (3H, m), 8.08-8.12(2H, m).
Mass (EI, m/z): 228(M⁺).

### Example 10: 2-Methyl-3,5,7,8-tetrahydropyrano[4,3-d]pyrimidin-4-one

The product was obtained in the same method as in Example 9 except that acetamidine hydrochloride was used instead of benzamidine hydrochloride.
¹H-NMR (CDCl₃) δ : 2.46(3H, s), 2.71(2H, t, J=5.6Hz), 3.97(2H, t, J=5.6Hz), 4.58(2H, s).
Mass (EI, m/z): 166(M⁺).

### Example 11: 2-Benzyl-3,5,7,8-tetrahydrothiopyrano[4,3-d]pyrimidin-4-one

The above-titled product was obtained in the same method as in Example 1 except that 2-phenylacetamidine was used instead of 4-trifluoromethylbenzamidine.
¹H-NMR (CDCl₃) δ : 2.87-2.92 (2H, m), 2.94-2.99(2H,m), 3.59 (2H, s), 3.97(2H,s), 7.27-7.39 (5H, m)
Mass (EI, m/z): 258(M⁺).

### Example 12: 2-(3-Nitrophenyl)-3,5,7,8-tetrahydrothiopyrano[4,3-d]pyrimidin-4-one

The above-titled product was obtained in the same method as in Example 1 except that 3-nitrobenzamidine was used instead of 4-trifluoromethylbenzamidine.
¹H-NMR (10%CD₃OD/CDCl₃) δ : 2.92-2.97(2H, m), 3.04-3.09(2H,m), 3.68 (2H, s), 7.73 (1H, t, J=8.0Hz), 8.39-8.43(2H, m), 8.95(1H, t, J=1.9Hz).
Mass (EI, m/z): 289(M⁺).

### Example 13: 2-(4-Fluorobenzyl)-3,5,7,8-tetrahydrothiopyrano[4,3-d]pyrimidin-4-one

The above-titled product was obtained in the same method as in Example 1 except that 2-(4-fluorophenyl)acetamidine was used instead of 4-trifluoromethyl-benzamidine.
¹H-NMR (CDCl₃) δ : 2.87-2.92(2H, m), 2.94-2.99(2H, m), 3.60(2H, s),3.93(2H, s), 7.04(2H, dd, J=8.5, 8.8Hz), 7.31(2H, dd, J=5.3, 8.5Hz).
Mass (EI, m/z): 276(M⁺).

### Example 14: 2-(3-Pyridyl)-3,5,7,8-tetrahydrothiopyrano[4,3-d]pyrimidin-4-one

The above-titled product was obtained in the same method as in Example 1 except that 3-amidinopyridine was used instead of 4-trifluoromethylbenzamidine.
¹H-NMR (10%CD₃OD/CDCl₃) δ : 2.94(2H, t, J=5.6Hz), 3.04(2H, t, J=5.6Hz), 3.68(2H, s), 7.49(1H, dd, J=5.1, 8.1Hz), 8.37(1H, ddd, J=1.9, 2.2, 8.1Hz), 8.75(1H, dd, J=1.9, 5.1Hz), 9.21(1H, d, J=2.2Hz).
Mass (EI, m/z): 245(M⁺).

### Preparation example 3: Methyl 2-(2-methoxycarbonylethylsulfanyl)benzoate

2-Mercaptobenzoic acid methyl ester (3.4 g) was dissolved in toluene (20 ml) and added with methyl acrylate (1.7 g). Triethylamine (12 ml) was added and the mixture was stirred at 80°C for 7 hours, and then returned to room temperature. The precipitate was dissolved in methanol (10 ml). The solution was added dropwise to water (1,000 ml), and the deposit was collected by filtration with a glass filter and dissolved in ethyl acetate. This solution was dried over anhydrous magnesium sulfate and evaporated under reduced pressure to obtain the above-titled product (4.4 g).
¹H-NMR (CDCl₃) δ : 2.72 (2H, t, J=7.6Hz), 3.22 (2H, t, J=7.6Hz), 3.72 (3H, s), 3.91 (3H, s), 7.18-7.22 (1H, m), 7.33-7.36 (1H, m), 7.44-7.50 (1H, m), 7.95-7.98 (1H, m).
Mass (TSP, m/z): 255(M+H)⁺.

### Example 15: 3-Methyl-2,10-dihydro-9-thia-2,4-diazaphenanthren-1-one

Methyl 4-oxo-thiochroman-3-carboxylate was synthesized in the same method as in Preparation example 1 except that methyl 2-(2-methoxycarbonyl-ethylsulfanyl)-benzoate obtained in Preparation example 3 was used instead of dimethyl 3,3'-thiodipropionate. The above-titled product was synthesized in the same method as in Example 2 except that methyl 4-oxotetrahydrothiopyran-3-carboxylate was used instead of methyl 4-oxothiochroman-3-carboxylate.
¹H-NMR, (DMSO) δ : 2.34 (3H, s), 3.83 (2H, s), 7.25-7.38 (3H, m), 8.13-8.17 (1H, m). Mass (EI, m/z): 230(M+).

### Preparation example 4: Methyl 3-methoxycarbonylmethylsulfanylpropionate

The product was obtained in the same method as in Preparation example 3 except that methyl mercaptoacetate was used instead of methyl 2-mercaptobenzoate.
¹H-NMR (CDCl₃) δ : 2.66 (2H, t, J=7.3Hz), 2.92 (2H, t, J=7.3Hz), 3.26 (2H, s), 3.71 (3H, s), 3.75 (3H, s).
Mass (EI, m/z): 192(M⁺).

### Example 16: 2-Methyl-6,7-dihydro-3H-thieno[3,2-d]pyrimidin-4-one

Methyl 3-oxotetrahydrothiophene-2-carboxylate was synthesized in the same method as in Preparation example 1 except that methyl 3-methoxycarbonylmethyl-sulfanylpropionate obtained in Preparation example 4 was used instead of dimethyl 3,3'-thiodipropionate. The above-titled product was synthesized in the same method as in Example 2 except that methyl 4-oxotetrahydrothiopyrane-3-carboxylate was used instead of methyl 3-oxotetrahydrothiophen-2-carboxylate.
¹H-NMR (DMSO) δ : 2.28 (3H, s), 3.92 (2H, t, J=3.2Hz), 4.06 (2H, t, J=3.2Hz).
Mass (EI, m/z): 168(M⁺).

### Example 17: 2-Phenyl-6,7-dihydro-3H-thieno[3,2-d]pyrimidin-4-one

The above-titled product was synthesized in the same method as in Example 16 except that acetamidine hydrochloride was used instead of benzamidine hydrochloride.
¹H-NMR (CDCl₃) δ: 4.16-4.19 (2H, m), 4.27-4.30 (2H, m), 7.51-7.62 (3H, m), 8.06-8.11 (2H, m).
Mass (TSP, m/z): 168(M+H)⁺.

### Example 18: 2-(3,5-Dimethyl-pyrazol-1-yl)-3,5,7,8-tetrahydrothiopyrano[4,3-d]-pyrimidin-4-one

The above-titled product was synthesized in the same method as in Example 1 except that 3,5-dimethylpyrazol-1-carboxamidine nitrate was used instead of 4-trifluoromethylbenzamidine, and ethane-1,2-diol was used instead of methanol.
¹H-NMR (CDCl₃) δ : 2.25 (3H, s), 2.64 (3H, s), 2.90 (4H, s), 3.64 (2H, s), 6.02 (1H, s). Mass (TSP, m/z): 263(M+H)⁺.

### Example 19: 6,7-Dihydro-3H-thieno[3,2-d]pyrimidin-4-one

The product was obtained in the same method as in Example 16 except that formamidine hydrochloride was used instead of acetamidine hydrochloride.
¹H-NMR (DMSO) δ : 3.32 (1H, s), 3.95 (2H, s), 4.11 (2H, s).
Mass (EI, m/z): 154(M⁺).

### Preparation example 5: 5-Methylene-4-oxo-tetrahydrothiopyrane-3-carboxylic acid methyl ester

Methyl 4-oxotetrahydrothiopyrane-3-carboxylate (1.7 g) was dissolved in acetic anhydride (10 ml), and the solution was added dropwise with N,N,N',N'-tetramethyldiaminomethane (2.7 ml) at room temperature, and then stirred at 60°C for 10 minutes. The mixture was returned to room temperature, then added with saturated aqueous sodium hydrogencarbonate (200 ml), and twice extracted with ethyl acetate (300 ml). The combined organic layer was dried over anhydrous magnesium sulfate and evaporated under reduced pressure. The residue obtained was purified by silica gel column chromatography to obtain the above-titled compound (921 mg).
¹H-NMR (CDCl₃) δ : 3.41 (2H, s), 3.49 (2H, s), 3.80 (3H, s), 5.33-5.35 (1H, m), 5.98 (1H, br s), 12.4 (1H, s).
Mass (EI, m/z): 186(M⁺).

### Example 20: 2-Methyl-8-methylene-3,5,7,8-tetrahydrothiopyrano[4,3-d]pyrimidin-4-one

The above-titled product was synthesized in the same method as in Example 2 except that 5-methylene-4-oxo-tetrahydrothiopyrane-3-carboxylic acid methyl ester obtained in Preparation example 5 was used instead of methyl 4-oxotetrahydrothiopyrane-3-carboxylate.
¹H-NMR (CDCl₃) δ : 2.46 (3H, s), 3.53 (2H, s), 3.74 (2H, s), 5.42 (1H, s), 6.40 (1H, s). Mass (EI, m/z): 194(M⁺).

### Preparation example 6: Ethyl 3-methylene-2-oxocyclohexanecarboxylate

The above-titled product was synthesized in the same method as in Preparation example 5 except that ethyl 2-oxocyclohexanecarboxylate was used instead of methyl 4-oxotetrahydrothiopyrane-3-carboxylate.
¹H-NMR (CDCl₃) δ : 1.31 (3H, t, J=7.0Hz), 1.69 (2H, quintet, J=6.1Hz), 2.36 (2H, t, J=6.1Hz), 2.42 (2H, m), 4.23 (2H, q, J=7.0Hz), 5,17 (1H, br s), 5.81 (1H,br s), 12.1 (1H, s).
Mass (EI, m/z): 182(M⁺).

### Example 21: (A) 2-Methyl-8-methylene-5,6,7,8-tetrahydro-3H-quinazolin-4-one and (B) 8-methoxymethyl-2-methyl-5,6,7,8-tetrahydro-3H-quinazolin-4-one

The aforementioned two compounds were obtained by carrying out the synthesis in the same method as in Example 2 except that ethyl 3-methylene-2-oxocyclohexanecarboxylate obtained in Preparation example 6 was used instead of methyl 4-oxotetrahydrothiopyrane-3-carboxylate.
(A) 2-Methyl-8-methylene-5,6,7,8-tetrahydro-3*H*-quinazolin-4-one
   ¹H-NMR (CDCl₃)δ : 1.81 (2H, quintet, J=6.1Hz), 2.46 (3H, s), 2.55 (2H, t, J=6.1Hz), 2.62 (2H, t, J=6.1Hz), 5.26 (1H, s), 6.25 (1H, s).
   Mass (EI, m/z): 176(M⁺).
(B) 8-Methoxymethyl-2-methyl-5,6,7,8-tetrahydro-3*H*-quinazolin-4-one
   ¹H-NMR (CDCl₃) δ : 1.66-1.96 (4H, m), 2.42 (3H, s), 2.44-2.53 (2H, m), 2.81-2.89 (1H, m), 3.37 (3H, s), 3.61 (1H, t, J=9.6Hz), 3.73 (1H, dd, J=3.6, 9.6Hz).
   Mass (EI, m/z): 208(M⁺).

### Example 22: (A) 2-Phenyl-8-methylene-5,6,7,8-tetrahydro-3H-quinazolin-4-one and (B) 2-phenyl-8-methoxymethyl-5,6,7,8-tetrahydro-3H-quinazolin-4-one

The aforementioned two compounds were obtained by carrying out the synthesis in the same method as in Example 21 except that benzamidine hydrochloride was used instead of acetamidine hydrochloride.
(A) 2-Phenyl-8-methylene-5,6,7,8-tetrahydro-3H-quinazolin-4-one
   ¹H-NMR (CDCl₃) δ : 1.87 (2H, quintet, J=6.2Hz), 2.62 (2H, t, J=6.2Hz), 2.72 (2H, t, J=6.2Hz), 5.33 (1H, br s), 6.46 (1H, br s), 7.50-7.58 (3H, m), 8.18-8.23 (2H, m).
   Mass (EI, m/z): 238(M⁺).
(B) 2-Phenyl-8-methoxymethyl-5,6,7,8-tetrahydro-3H-quinazolin-4-one
   ¹H-NMR (CDCl₃) δ : 1.65-1.80 (1H, m), 1.84-1.98 (3H, m), 2.57-2.63 (2H, m), 2.93-3.02 (1H, m), 3.40 (3H, s), 3.73 (1H, t, J=8.6Hz), 3.90 (1H, dd, J=3.4, 8.6Hz), 7.48-7.56 (3H, m), 8.18-8.23 (2H, m).
   Mass (EI, m/z): 270(M⁺).

### Example 23: 2-Phenyl-8-methylene-3,5,7,8-tetrahydrothiopyrano[4,3-d]pyrimidin-4-one

The product was obtained in the same method as in Example 20 except that acetamidine hydrochloride was used instead of benzamidine hydrochloride.
¹H-NMR (CDCl₃) δ : 3.60 (2H, s), 3.84 (2H, s), 5.49 (1H, d, J=1.5Hz), 6.60 (1H, d, J=1.5Hz), 7.51-7.60 (3H, m), 8.16-8.21 (2H, m).
Mass (EI, m/z): 256(M⁺).

### Example 24: 8-Hydroxymethyl-2-methyl-5,6,7,8-tetrahydro-3H-quinazolin-4-one

2-Methyl-8-methylene-5,6,7,8-tetrahydro-3*H*-quinazolin-4-one (70 mg) obtained in Example 21 was dissolved in tetrahydrofuran (2.0 ml), and the solution was added with the Wilkinson's catalyst (5.7 mg) and diethoxymethylsilane (0.32 ml), and heated under reflux for one day. The mixture was returned to room temperature, then added with methanol (2.0 ml), potassium hydrogencarbonate (46 mg) and 30% aqueous hydrogen peroxide (2.0 ml), and heated under reflux for 6 hours. After the resulting mixture was returned to room temperature, methanol (10 ml) was added to the mixture and the precipitate was removed, and then the mixture was evaporated. The obtained residue was purified by silica gel column chromatography to obtain the above-titled product (9.5 mg).
¹H-NMR (CD₃OD) δ : 1.64-1.73 (1H, m), 1.80-1.90 (3H, m), 2.34 (3H, s), 2.38-2.44 (2H, m), 2.68-2.74 (1H, m), 3.73 (1H, dd, J=7.0, 11Hz), 3.82 (1H, dd, J=4.8, 11Hz).
Mass (EI, m/z): 194(M⁺).

### Example 25: 2-Thiophen-2-yl-3,5,7,8-tetrahydrothiopyrano[4,3-d]pyrimidin-4-one

The product was obtained in the same method as in Example 1 except that 2-amidinothiophene hydrochloride was used instead of 4-trifluoromethylbenzamidine.
¹H-NMR (CDCl₃) δ : 2.92 (2H, t, J=5.7Hz), 3.01 (2H, t, J=5.7Hz), 3.71 (2H, br s), 7.18 (1H, dd, J=3.9, 5.1Hz), 7.56 (1H, dd, J=1.2, 5.1Hz), 8.11 (1H, dd, J=1.2, 3.9Hz).
Mass (EI, m/z): 250(M⁺).

### Preparation example 7: 2-(4-Chlorophenoxv)acetamidine

4-Chlorophenoxyacetonitrile (5.0 g) was dissolved in ethanol (30 ml), and the solution was bubbled with hydrogen chloride gas at -20°C for 15 minutes, and then the reaction system was sealed and allowed to stand at the same temperature overnight. The mixture was returned to room temperature and then added dropwise to diethyl ether (1,000 ml), and the resulting white precipitate was collected by filtration with a glass filter and suspended in diethyl ether (50 ml). The suspension was bubbled with ammonia gas at -20°C for 20 minutes, and the mixture was added with ethanol (50 ml) and heated under reflux for 2 hours. After the mixture was returned to room temperature, the precipitate was collected by filtration, and the filtrate was added dropwise to diethyl ether (500 ml). The white precipitate deposited was collected by filtration with a glass filter and dried to obtain the above-titled product (5.6 g).
¹H-NMR (CD₃OD) δ: 4.94 (2H, s), 7.02-7.07 (2H, m), 7.32-7.36 (2H, m).
Mass (EI, m/z): 184(M⁺).

### Example 26: 2-(4-Chlorophenoxymethyl)-3,5,7,8-tetrahydrothiopyrano[4,3-d]-pyrimidin-4-one

The product was obtained in the same method as in Example 1 except that 2-(4-chlorophenoxy)acetamidine obtained in Preparation example 7 was used instead of 4-trifluoromethylbenzamidine.
¹H-NMR (DMSO) δ : 2.74-2.80 (2H, m), 2.83-2.87 (2H, m), 3.46 (2H, s), 4.89 (2H, s), 7.00-7.07 (2H, m), 7.32-7.38 (2H, m).
Mass (EI, m/z): 308(M⁺).

### Preparation example 8: Heptaneamidine

The product was obtained in the same method as in Preparation example 7 except that n-heptanenitrile was used instead of 4-chlorophenoxyacetonitrile.
¹H-NMR (CD₃OD) δ : 0.92 (3H, t, J=7.0Hz), 1.30-1.45 (6H, m), 1.65-1.74 (2H, m), 2.42-2.48 (2H, m).
Mass(TSP, m/z): 129(M+H)⁺.

### Example 27: 2-Hexyl-3,5,7,8-tetrahydrothiopyrano[4,3-d]pyrimidin-4-one

The product was obtained in the same method as in Example 1 except that heptaneamidine was used instead of 4-trifluoromethylbenzamidine.
¹H-NMR (CDCl₃) δ : 0.89 (3H, t, J=7.6Hz), 1.28-1.42 (6H, m), 1.74 (2H, quintet, J=7.6Hz), 2.58-2.64 (2H, m), 2.88-2.93 (4H, m), 3.61 (2H, s).
Mass (EI, m/z): 253(M⁺).

### Preparation example 9: 2-Phenoxyacetamidine

The product was obtained in the same method as in Preparation example 7 except that phenoxyacetonitrile was used instead of 4-chlorophenoxyacetonitrile.
¹H-NMR (CD₃OD) δ : 4.86 (2H, s), 7.01-7.08 (3H, m), 7.31-7.37 (2H, m).
Mass(FAB, m/z): 129(M+H)⁺.

### Example 28: 2-Phenoxymethyl-3,5,7,8-tetrahydrothiopyrano[4,3-d]pyrimidin-4-one

The product was obtained in the same method as in Example 1 except that 2-phenoxyacetamidine obtained in Preparation example 9 was used instead of 4-trifluoromethylbenzamidine.
¹H-NMR (DMSO)δ : 2.74-2.81 (2H, m), 2.83-2.88 (2H, m), 3.47 (2H, s), 4.87 (2H, s), 6.95-7.04 (3H, m), 7.28-7.34 (2H, m).
Mass (EI, m/z): 275(M⁺).

### Preparation example 10: 4-Fluorophenoxyacetonitrile

4-Fluorophenol (5.7 g) was dissolved in acetone (50 ml) and added with potassium carbonate (14 g). A solution of chloroacetonitrile (3.2 ml) in acetone (50 ml) was added dropwise at room temperature over 1.5 hours, and the mixture was heated under reflux for one day. The mixture was returned to room temperature, then added with water (400 ml), and extracted with diethyl ether (600 ml). The organic layer was washed successively with 1 N aqueous sodium hydroxide (300 ml), saturated aqueous sodium thiosulfate (200 ml) and water (200 ml), then dried over anhydrous magnesium sulfate, and evaporated under reduced pressure to obtain the above-titled product (8.5 g).
¹H-NMR (CD₃OD) δ : 4.73 (2H, s), 6.93-6.99 (2H, m), 7.01-7.08 (2H, m).
Mass (EI, m/z): 151(M⁺).

### Preparation example 11: 2-(4-Fluorophenoxy)acetamidine

The product was obtained in the same method as in Preparation example 7 except that 4-fluorophenoxyacetonitrile obtained in Preparation example 10 was used instead of 4-chlorophenoxyacetonitrile.
¹H-NMR (DMSO) δ : 4.92 (2H, s), 7.03-7.11 (4H, m).
Mass (EI, m/z): 168(M⁺).

### Example 29: 2-(4-Fluorophenoxvmethvl)-3,5,7,8-tetrahydrothiopyrano[4,3-d]-pyrimidin-4-one

The product was obtained in the same method as in Example 1 except that 2-(4-fluorophenoxy)acetamidine obtained in Preparation example 11 was used instead of 4-trifluoromethylbenzamidine.
¹H-NMR (DMSO) δ : 2.77 (2H, t, J=5.8Hz), 2.85 (2H, t, J=5.8Hz), 3.47 (2H, s), 4.87 (2H, s), 7.02-7.07 (2H, m), 7.11-7.18 (2H, m).
Mass(TSP, m/z): 293(M+H)⁺.

### Preparation example 12: 3-Fluorophenoxyacetonitrile

The product was obtained in the same method as in Preparation example 10 except that 3-fluorophenol was used instead of 4-fluorophenol.
¹H-NMR, (CDCl₃) δ : 4.76 (2H, s), 6.72 (1H, dt, J=2.5, 10Hz), 6.78 (1H,dd, J=2.5, 8.3Hz), 6.82 (1H, ddd, J=0.8, 2.5, 8.3Hz), 7.31 (1H, dt, J=6.6, 8.3Hz).
Mass (EI, m/z): 151(M⁺).

### Preparation example 13: 2-(3-Fluorophenoxy)acetamidine

The product was obtained in the same method as in Preparation example 7 except that 3-fluorophenoxyacetonitrile was used instead of 4-chlorophenoxy-acetonitrile.
¹H-NMR (DMSO) δ : 5.01 (2H, s), 6.85-6.93 (3H, m), 7.36-7.43 (1H, m).
Mass (EI, m/z): 168(M+).

### Example 30: 2-(3-Fluorophenoxymethyl)-3,5,7,8-tetrahydrothiopyrano[4,3-d]-pyrimidin-4-one

The product was obtained in the same method as in Example 1 except that 2-(3-fluorophenoxy)acetamidine was used instead of 4-trifluoromethylbenzamidine.
¹H-NMA (DMSO) δ : 2.78 (2H, t, J=5.6Hz), 2.85 (2H, t, J=5.6Hz), 3.47 (2H, s), 4.91 (2H, s), 6.82 (1H, ddt, J=1.0, 2.5, 8.2Hz), 6.87 (1H, ddd, J=1.0, 2.5, 8.2Hz), 6.92 (1H, dt, J=2.5, 11Hz), 7.34 (1H, dt, J=7.0, 8.2Hz).
Mass (TSP, m/z): 293(M+H)⁺.

### Preparation example 14: 2-Fluorophenoxyacetonitrile

The product was obtained in the same method as in Preparation example 10 except that 2-fluorophenol was used instead of 4-fluorophenol.
¹H-NMR (CDCl₃) δ : 4.83 (2H, s), 7.06-7.18 (4H, m).
Mass (EI, m/z): 151(M⁺).

### Preparation example 15: 2-(2-Fluorophenoxy)acetamidine

The product was obtained in the same method as in Preparation example 7 except that 2-fluorophenoxyacetonitrile obtained in Preparation example 14 was used instead of 4-chlorophenoxyacetonitrile.
¹H-NMR (DMSO) δ : 5.06 (2H, s), 7.04-7.10 (1H, m), 7.06-7.22 (2H, m), 7.27-7.33 (1H, m).
Mass (EI, m/z): 168(M⁺).

### Example 31: 2-(2-Fluorophenoxymethyl)-3,5,7,8-tetrahydrothiopyrano[4,3-d]-pyrimidin-4-one

The product was obtained in the same method as in Example 1 except that 2-(2-fluorophenoxy)acetamidine obtained in Preparation example 15 was used instead of 4-trifluoromethylbenzamidine.
¹H-NMR (DMSO) δ : 2.77 (2H, t, J=5.6Hz), 2.85 (2H, t, J=5.6Hz), 3.47 (2H, s), 4.94 (2H, s), 6.96-7.02 (1H, m), 7.11-7.16 (1H, m), 7.17-7.27 (2H,m).
Mass (EI, m/z): 292(M⁺).

### Preparation example 16: 4-Hydroxybutyronitrile

3-Bromopropanol (1.4 g) was dissolved in methanol (50 ml), and added with sodium cyanide (2.6 g) dissolved in water (7.0 ml). The mixture was stirred at 50°C for 7 hours, then returned to room temperature, and evaporated under reduced pressure. The residue was added with water (100 ml) and extracted twice with ethyl acetate (150 ml), and the combined organic layer was dried over anhydrous magnesium sulfate. Evaporation was carried out under reduced pressure to obtain the above-titled product (534 mg).
¹H-NMR (CDCl₃) δ : 1.91 (2H, tt, J=5.8, 7.0Hz), 2.51 (2H, t, J=7.0Hz), 2.51 (2H, t, J=5.8Hz).
Mass (EI, m/z): 85(M⁺).

### Example 32: 2-(3-Hydroxypropyl)-3,5,7,8-tetrahydrothiopyrano[4,3-d]-pyrimidin-4-one

4-Hydroxybutylamidine was obtained in the same procedures as in Preparation example 7 except that 4-hydroxybutyronitrile obtained in Preparation example 16 was used instead of 4-chlorophenoxyacetonitrile. Successively, the above-titled product was synthesized in the same method as in Example 1 except that 4-hydroxybutylamidine previously obtained was used instead of 4-trifluoromethyl-benzamidine.
¹H-NMR (CDCl₃) δ : 2.02 (2H, quintet, J=5.6Hz), 2.82-2.96 (6H, m), 3.60 (2H, s), 3.73 (2H, t, J=5.6Hz).
Mass (EI, m/z): 226(M⁺).

### Example 33: 2,3,3a,4,8,9-Hexahydro-1H,6H-7-thia-4,9b-diazacyclopenta[a]-naphthalene-5-one

2-(3-Hydroxypropyl)-3,5,7,8-tetrahydrothiopyrano[4,3-d]pyrimidin-4-one (116 mg) was dissolved in a mixed solvent of methylene chloride/tetrahydrofuran (5.0 ml/5.0 ml), and added with methanesulfonyl chloride (0.040 ml). Triethylamine (0.072 ml) was added at room temperature, and the mixture was stirred at the same temperature for 30 minutes. Water (50 ml) was added to stop the reaction, and the mixture was extracted twice with methylene chloride (50 ml). The combined organic layer was dried over anhydrous magnesium sulfate and evaporated under reduced pressure. The obtained residue was purified by silica gel column chromatography to obtain the above-titled product (24 mg).
¹H-NMR (CDCl₃) δ : 2.28 (2H, quintet, J=7.7Hz), 2.77-2.82 (2H, m), 2.89 (2H, t, J=5.6Hz), 3.06 (2H, t, J=7.7Hz), 3.61 (2H, s), 4.02 (2H, t, J=7.7Hz).
Mass (EI, m/z): 208(M⁺).

### Example 34: 2-Methyl-8-propylidene-3,5,7,8-tetrahydrothiopyrano[4,3-d]-pyrimidin-4-one

Diisopropylamine (2.3 ml) was dissolved in tetrahydrofuran (30 ml) under argon atmosphere, and the solution was added dropwise with a n-butyllithium-hexane solution (1.53 M, 10 ml) at -10°C. The mixture was stirred at the same temperature for 30 minutes, then added dropwise with a tetrahydrofuran solution (6.0 ml) of 4-oxothiane (1.7 g) at -78°C, stirred at the same temperature for 10 minutes, and further added dropwise with trimethylsilyl chloride (2.3 ml). The resulting mixture was stirred for 15 minutes, then poured into water (500 ml) to stop the reaction, and extracted twice with diethyl ether (500 ml). The combined organic layer was dried over anhydrous magnesium sulfate and evaporated under reduced pressure. The obtained residue was purified by silica gel column chromatography to obtain (3,6-dihydro-2H-thiopyran-4-yloxy)trimethylsilane (1.9 g).

Propionaldehyde (0.30 ml) was dissolved in methylene chloride (26 ml) under an atmosphere of argon, and the solution was added with titanium tetrachloride (0.36 ml) at -78°C, and added dropwise with a solution of(3,6-dihydro-2*H*-thiopyran-4-yloxy)trimethylsilane (617 mg) in methylene chloride (6.5 ml) which was previously obtained. The mixture was stirred at the same temperature for 10 minutes, then added with 5% aqueous sodium hydrogencarbonate (30 ml) to stop the reaction and further added with water (200 ml), and then extracted with diethyl ether (200 ml) twice. The resulting organic layer was dried over anhydrous magnesium sulfate and evaporated under reduced pressure. The obtained residue was purified by silica gel column chromatography to obtain 3-(1-hydroxypropyl)-tetrahydrothiopyran-4-one (243 mg).

The resulting 3-(1-hydroxypropyl)-tetrahydrothiopyran-4-one (241 mg) was dissolved in methylene chloride (13 ml), and the solution was added with ethyl vinyl ether (0.27 ml) and a catalytic amount of pyridinium p-toluenesulfonate, and stirred at room temperature for 12 hours. The mixture was poured into 5% aqueous sodium hydrogencarbonate (30 ml) to stop the reaction, added with water (50 ml), and extracted twice with methylene chloride (100 ml). The combined organic layer was dried over anhydrous magnesium sulfate and evaporated under reduced pressure. The residue obtained was purified by silica gel column chromatography to obtain 3-[1-(1-ethoxyethoxy)propyl]tetrahydrothiopyran-4-one (267 mg).

Diisopropylamine (0.20 ml) was dissolved in tetrahydrofuran (5.0 ml) under an atmosphere of argon, and added dropwise with a n-butyllithium-hexane solution (1.53 M, 0.75 ml) at -10°C. The mixture was stirred at the same temperature for 30 minutes, and then added dropwise at -78°C with a tetrahydrofuran solution (5.0 ml) of 3-[1-(1-ethoxyethoxy)propyl]tetrahydrothiopyran-4-one (267 mg), which was previously obtained. The resulting mixture was stirred at the same temperature for 10 minutes, then added with hexamethylphosphoric triamide (0.080 ml) and cyanoacetic acid methyl ester (0.11 ml) at -10°C, and stirred for 1 hour. Water (50 ml) was added to stop the reaction, and the mixture was extracted twice with methylene chloride (100 ml). The combined organic layer was dried over anhydrous magnesium sulfate and evaporated under reduced pressure. The residue obtained was dissolved in methanol (5.0 ml), added with acetamidine hydrochloride (105 mg) and potassium carbonate (281 mg), and heated under reflux for 2 hours. The mixture was returned to room temperature and purified by silica gel column chromatography to obtain the above-titled product (31 mg).
¹H-NMR (DMSO) δ : 1.02 (3H, t, J=7.5Hz), 2.21 (2H, q, J=7.5Hz), 2.22 (3H, s), 3.50 (2H, s), 3.55 (2H, s), 6.91 (1H, t, J=7.5Hz).
Mass (FAB, m/z): 293(M+H)+.

### Example 35: 8-Ethylidene-2-methyl-3,5,7,8-tetrahydrothiopyrano[4,3-d]-pyrimidin-4-one

The product was obtained in the same method as in Example 34 except that acetaldehyde was used instead of propionaldehyde.
¹H-NMR (CDCl₃) δ : 1.90 (3H, d, J=7.3Hz), 2.44 (3H, s), 3.52 (2H, s), 3.72 (2H, s), 7.16 (1H, q, J=7.3Hz).
Mass (EI, m/z): 208(M⁺).

### Example 36: 2-Methyl-8-(2-methylpropylidene)-3,5,7,8-tetrahydrothiopyrano[4,3,-d]-pyrimidin-4-one

The product was obtained in the same method as in Example 34 except that 2-methylpropionaldehyde was used instead of propionaldehyde.
¹H-NMR (CDCl₃) δ : 1.10 (6H, d, J=6.6Hz), 2.44 (3H, s), 2.68-2.79 (1H, m), 3.53 (2H, s), 3.72 (2H, s), 6.93 (1H, d, J=9.7Hz).
Mass (EI, m/z): 236(M⁺).

### Example 37: 8-Cyclohexylmethylene-2-methyl-3,5,7,8-tetrahydrothiopyrano[4,3-d]-pyrimidin-4-one

The product was obtained in the same method as in Example 34 except that cyclohexanecarboxyaldehyde was used instead of propionaldehyde.
¹H-NMR (CDCl₃) δ: 1.12-1.40 (6H, m), 1.66-1.82 (4H, m), 2.40 (3H, s), 2.65 (1H, d, J=9.3Hz), 3.52 (2H, s), 3.74 (2H, s), 6.92 (1H, d, J=9.3Hz).
Mass (EI, m/z): 276(M⁺).

### Example 38: 2-(3-Hydroxypentyl)-3,5,7,8-tetrahydrothiopyrano[4,3-d]-pyrimidin-4-one

3-Chloropentanol (2.5 g) was dissolved in methanol (50 ml), and the solution was added with potassium iodide (3.3 g) and stirred for 30 minutes, and then added with sodium cyanide (1.1 g) dissolved in water (10 ml). The mixture was heated under reflux for 17.5 hours, then returned to room temperature, and evaporated under reduced pressure. Water (100 ml) was added, and the mixture was extracted twice with ethyl acetate (100 ml). The combined organic layer was dried over anhydrous magnesium sulfate. The solvent was evaporated under reduced pressure to obtain 4-hydroxypentyronitrile (972 mg), and 6-hydroxyhexaneamidine was obtained in the same procedures as in Preparation example 7 except that 4-hydroxypentyronitrile was used instead of 4-chlorophenoxyacetonitrile. Successively, the above-titled product was synthesized in the same method as in Example 1 except that 6-hydroxyhexane-amidine previously obtained was used instead of 4-trifluoromethylbenzamidine.
¹H-NMR (CDCl₃) δ : 1.43-1.53 (2H, m), 1.57-1.67 (2H, m), 1.81 (2H, quintet, J=7.5Hz) 2.65 (2H, d, J=7.5Hz), 2.86-2.96 (4H, m), 3.60 (2H, s), 3.67 (2H, t, J=6.3Hz).
Mass (EI, m/z): 254(M⁺).

### Example 39: 8-(2-Methoxyethylidene)-2-methyl-3,5,7,8-tetrahydrothiopyrano-[4,3-d]pyrimidin-4-one

Methoxyacetic acid methyl ester (536 mg) was dissolved in methylene chloride (10 ml) under argon atmosphere, and added dropwise with a diisobutylaluminium hydride-hexane solution (0.9 M, 5.5 ml) at -78°C. The mixture was stirred at the same temperature for 1 hour, and then added with saturated aqueous ammonium chloride (1.0 ml) to stop the reaction. The mixture was dried over anhydrous magnesium sulfate, and then evaporated under reduced pressure to obtain methoxyacetaldehyde, and the above-titled product (2.4 mg) was synthesized in the same method as in Example 34 except that methoxyacetaldehyde was used instead of propionaldehyde.
¹H-NMR (CDCl₃) δ : 2.43 (3H, s), 3.40 (3H, s), 3.50 (2H, s), 3.71 (2H, s), 4.22 (2H, d, J=6.3Hz), 7.14 (1H, t, J=6.3Hz).
Mass (TSP, m/z): 239(M+H)⁺.

### Example 40: 8-(2-Methoxypropylidene)-2-methyl-3,5,7,8-tetrahydrothiopyrano-[4,3-d]pyrimidin-4-one

The product was obtained in the same method as in Example 39 except that methyl 3-methoxypropionate was used instead of methyl methoxyacetate.
¹H-NMR (CDCl₃) δ : 2.43 (3H, s), 2.55 (2H, q, J=7.0Hz) 3.37 (3H, s), 3.53 (2H, s), 3.54 (2H, t, J=7.0Hz), 3.71 (2H, s), 7.08 (1H, t, J=7.0Hz).
Mass (TSP, m/z): 253(M+H)⁺.

### Example 41: 8-(2-Hydroxyethylidene)-2-methyl-3,5,7,8-tetrahydrothiopyrano[4,3-d]-pyrimidin-4-one

8-[2-(*tert*-Butyldiphenylsilyloxy)ethylidene]-2-methyl-3,5,7,8-tetrahydro-thiopyrano[4,3-d]pyrimidin-4-one was synthesized in the same method as in Example 39 except that (*tert*-butyldiphenylsilyloxy)acetic acid methyl ester was used instead of methoxyacetic acid methyl ester. The resulting compound was dissolved in tetrahydrofuran under argon atmosphere, and the solution was added with a tetrabutylammonium fluoride-tetrahydrofuran solution at room temperature, stirred for 18 hours, and then evaporated under reduced pressure. The residue was purified by silica gel column chromatography to obtain the above-titled product.
¹H-NMR (DMSO) δ : 2.26 (3H, s), 3.48 (2H, s), 3.55 (2H, s), 4.22 (2H, t, J=5.8Hz), 6.96 (1H, t, J=5.8Hz).
Mass (TSP, m/z): 225(M+H)⁺.

### Example 42: 2-(2-Chlorophenoxvmethvl)-3,5,7,8-tetrahydrothiopyrano[4.3-dl-pyrimidin-4-one

The above-titled product was obtained in the same method as in Example 1 except that 2-(2-chlorophenoxy)acetamidine was used instead of 4-trifluoromethyl-benzamidine.
¹H-NMR (CDCl₃) δ: 2.86-2.96(4H, m), 3.62(2H, s ), 5.03-5.08(2H, m), 6.93-7.06(2H, m), 7.22-7.29(1H, m), 7.38-7.45(1H, m).
Mass (EI, m/z): 308(M⁺).

### Example 43: 2-(2-tert-Butyl)-3,5,7,8-tetrahydrothiopyrano[4,3-d]pyrimidin-4-one

The above-titled product was obtained in the same method as in Example 1 except that 2,2-dimethylpropionamidine was used instead of 4-trifluoromethyl-benzamidine.
¹H-NMR (CDCl₃) δ : 1.36(9H, s ), 2.85-2.96(4H, m), 3.61(2H, s).
Mass (EI, m/z): 224(M⁺).

### Example 44: 2-(2-Pyrazin-2-yl)-3,5,7,8-tetrahydrothiopyrano[4,3-d]pyrimidin-4-one

The above-titled product was obtained in the same method as in Example 1 except that pyrazin-2-carboxyamidine was used instead of 4-trifluoromethylbenzamidine.
¹H-NMR (CDCl₃) δ : 2.95(2H, t, J=5.9Hz), 3.05(2H, t,J=5.9Hz), 3.72(2H, s), 8.62(1H, dd, J=1.4,2.5Hz), 8.78(1H, d, J=2.5Hz), 9.64(1H, dd, J=1.4Hz).
Mass (EI, m/z): 246(M⁺).

### Example 45: 2-(5-Trifluoromethylpyridin-2-yl)-3,5,7,8-tetrahydrothiopyrano[4,3-d]-pyrimidin-4-one

The above-titled product was obtained in the same method as in Example 1 except that 5-trifluoromethylpyridin-2-carboxyamidine was used instead of 4-trifluoromethylbenzamidine.
¹H-NMR (CDCl₃) δ : 2.94(2H, t, J=5.8Hz), 3.05(2H, t,J=5.8Hz), 3.72(2H, s), 8.13(1H, dd, J=8.1Hz), 8.57(1H, d, J= 8.1Hz), 8.92(1H, s ), 10.86(1H, brs).
Mass (EI, m/z): 313(M⁺).

### Example 46: 2-(4-Chlorophenyl)-3,5,7,8-tetrahydrothiopyrano[4,3-d]pyrimidin-4-one

The above-titled product was obtained in the same method as in Example 1 except that 4-chlorobenzamidine was used instead of 4-trifluoromethylbenzamidine.
¹H-NMR (CDCl₃) δ : 2.93(2H, t, J=5.9Hz), 3.03(2H, t,J=5.9Hz), 3.67(2H, s), 7.50(2H, d, J=8.9Hz), 7.96(2H, d, J=8.9Hz).
Mass (EI, m/z): 278(M⁺).

### Example 47: 2-(2-Isopropyl)-3,5,7,8-tetrahydrothiopyrano[4.3-d]pyrimidin-4-one

The above-titled product was obtained in the same method as in Example 1 except that isobutylamidine was used instead of 4-trifluoromethylbenzamidine.
¹H-NMR (CDCl₃) δ : 1.32(6H, d, J=7.1Hz), 2.82-2.96(5H, m), 3.62(2H, s).
Mass (EI, m/z): 210(M⁺).

### Example 48: (A) 2-Methyl-3,5,7,8-tetrahydrothiopyrano[4,3-d]pyrimidin-4-one-6-oxide and (B) 2-methyl-3,5,7,8-tetrahydrothiopyrano[4,3-d]pyrimidin-4-one-6.6-dioxide

A solution of 2-methyl-3,5,7,8-tetrahydrothiopyrano[4,3-d]pyrimidin-4-one (185 mg), synthesized in Example 2, in methylene chloride (4 ml) was added with m-chloroperbenzoic acid (289 mg) and stirred for 20 hours. The solvent was evaporated, and the residue was purified by silica gel column chromatography to obtain the desired products (A) (149 mg) and (B) (77 mg).
(A) 2-Methyl-3,5,7,8-tetrahydrothiopyrano[4,3-d]pyrimidin-4-one-6-oxide
   ¹H-NMR (CDCl₃) δ : 2.44(3H, s), 2.78-2.98(2H, m), 3.28-3.45(2H, m), 3.62(1H, d, J=17.3Hz), 3.99(1H, d, J=17.3Hz).
   Mass (EI, m/z): 198(M⁺).
(B) 2-Methyl-3,5 ,7,8-tetrahydrothiopyrano[4,3-d)pyrimidin-4-one-6,6-dioxide
   ¹H-NMR (10% CD₃OD/CDCl₃) δ : 2.41(3H, s), 3.30(4H, s), 4.06(2H, s).
   Mass (EI, m/z): 214(M⁺).

### Example 49: 2-Phenylsulfanylmethyl-3,5,7,8-tetrahydrothiopyrano[4,3-d]pyrimidin-4-one

The above-titled product was obtained in the same method as in Example 1 except that 2-phenylsulfanylacetamidine was used instead of 4-trifluoromethyl-benzamidine,
¹H-NMR (CDCl₃) δ : 2.88(4H, brs), 3.58(2H, s), 4.00(2H, s), 7.17-7.30(3H, m), 7.37-7.42(2H, m).
Mass (EI, m/z): 290(M⁺).

### Example 50: 2-(4-Chlorophenylsulfanylmethyl)-3,5,7,8-tetrahydrothiopyrano[4,3-d]-pyrimidin-4-one

The above-titled product was obtained in the same method as in Example 1 except that 4-chlorophenylsulfanylacetamidine was used instead of 4-trifluoromethyl-benzamidine.
¹H-NMR (10%CD₃OD/CDCl₃) δ : 2.85(4H, brs), 3.57(2H, s), 3.94(2H, s), 7.25-7.29(2H, m), 7.31-7.35(2H, m).
Mass (EI, m/z): 324(M⁺).

### Example 51: 2-(Pyridin-4-yl)-3,5,7,8-tetrahydrothiopyrano[4,3-d]pyrimidin-4-one

The above-titled product was obtained in the same method as in Example 1 except that isonicotinacetamidine was used instead of 4-trifluoromethylbenzamidine.
¹H-NMR (10%CD₃OD/CDCl₃) δ : 2.95(2H, t, J=5.6Hz), 3.12(2H, t, J=5.6Hz), 3.68(2H, s), 7.98(1H, d, J=6.3Hz), 8.77 (1H, d, J=6.3Hz).
Mass (EI, m/z): 245(M⁺).

### Example 52: 2-(Pyridin-2-yl)-3,5,7,8-tetrahydrothiopyrano[4,3-d]pyrimidin-4-one

The above-titled product was obtained in the same method as in Example 1 except that pyridin-2-carboxyamidine was used instead of 4-trifluoromethyl-benzamidine.
¹H-NMR (CDCl₃) δ : 2.93(2H, t, J=5.6Hz), 3.03(2H, t, J=5.6Hz), 3.71(2H, s), 7.47(1H, ddd, J=1.1,4.9 and 7.5Hz), 7.88(1H, dd, J=1.7,7.5Hz), 8.41(1H, d, J=8.0Hz), 8.64(1H, d, J=4.4Hz).
Mass (EI, m/z): 245(M⁺).

### Preparation example 17: 3-Phenylpropionamidine

The above-titled product was obtained in the same method as in Preparation example 7 except that 3-phenylpropionitrile was used instead of 4-chlorophenoxy-acetonitrile.
¹H-NMR (DMSO-d₆) δ : 2.68(2H, t, J=7.5Hz), 2.93(2H, t, J=7.5Hz), 7.19-7.27(3H, m), 7.28-7.34(2H, m).
Mass (EI, m/z): 148(M⁺).

### Example 53: 2-(Phenethyl)-3,5,7,8-tetrahydrothiopyrano[4,3-d]pyrimidin-4-one

The above-titled product was obtained in the same method as in Example 1 except that 3-phenylpropionamidine obtained in Preparation example 17 was used instead of 4-trifluoromethylbenzamidine.
¹H-NMR (CDCl₃) δ: 2.88-2.99(6H, m), 3.04-3.10(2H, m), 3.68(2H, s), 7.18-7.33(5H, m).
Mass (EI, m/z): 272(M⁺).

### Preparation example 18: 2-(3-Chlorophenoxy)acetamidine

The above-titled product was obtained in the same method as in Preparation example 7 except that 2-(3-chlorophenoxy)acetonitrile was used instead of 4-chlorophenoxyacetonitrile.
¹H-NMR (DMSO-d₆) δ : 4.96(2H, s), 6.96-7.00(1H, m), 7.09-7.13(2H, m),7.35-7.41(1H, m).
Mass (EI, m/z): 184(M⁺).

### Example 54: 2-(3-Chlorophenoxymethyl)-3,5,7,8-tetrahydrothiopyrano[4,3-d]-pyrimidin-4-one

The above-titled product was obtained in the same method as in Example 1 except that 2-(3-chlorophenoxy)acetamidine obtained in Preparation example 18 was used instead of 4-trifluoromethylbenzamidine.
¹H-NMR (10%CD₃OD/CDCl₃) δ : 2.88-2.98(4H, m), 3.62(2H, s), 4.95(2H, s), 6.86-6.91(1H, m),7.01-7.07(2H, m), 7.27(1H, t, J=8.2Hz).
Mass (EI, m/z): 308(M⁺).

### Preparation example 19: 2-(2-Methylphenoxy)acetamidine

The above-titled product was obtained in the same method as in Preparation example 7 using 2-(2-methylphenoxy)acetonitrile instead of 4-chlorophenoxy-acetonitrile.
¹H-NMR (DMSO-d₆) δ : 4.92(2H, s), 6.85(1H, d, J=8.5Hz), 6.91-6.96(1H, m),7.16-7.22(2H, m), 9.15(3H, brs).
Mass (EI, m/z): 164(M⁺).

### Example 55: 2-(2-Methylphenoxymethyl)-3,5,7,8-tetrahydrothiopyrano[4,3-d]-pyrimidin-4-one

The above-titled product was obtained in the same method as in Example 1 except that 2-(2-methylphenoxy)acetamidine obtained in Preparation example 19 was used instead of 4-trifluoromethylbenzamidine.
¹H-NMR (CDCl₃) δ : 2.33(3H, s), 2.88-2.97(4H, m), 3.64(2H, s), 4.98(2H, s), 6.78(1H, d, J=7.6Hz), 6.98(1H, t, J=7.6Hz), 7.15-7.23(2H, m).
Mass (EI, m/z): 288(M⁺).

### Preparation example 20: 2-(2-Bromophenoxy)acetamidine

The above-titled product was obtained in the same method as in Preparation example 7 using 2-(2-bromophenoxy)acetonitrile instead of 4-chlorophenoxy-acetonitrile.
¹H-NMR (DMSO-d₆) δ : 4.99(2H, s), 7.03(1H, ddd, J=1.2, 7.6, 7.8Hz), 7.11(1H, dd, J=1.2, 8.3Hz), 7.42(1H, ddd, J=1.7, 7.6, 8.3Hz), 7.66(1H, dd, J=1.7, 7.8Hz).
Mass (FAB, m/z): 229(M+1),231.

### Example 56: 2-(2-Bromophenoxymethyl)-3,5,7,8-tetrahydrothiopyrano[4,3-dl-pyrimidin-4-one

The above-titled product was obtained in the same method as in Example 1 except that 2-(2-bromophenoxy)acetamidine obtained in Preparation example 20 was used instead of 4-trifluoromethylbenzamidine.
¹H-NMR (CDCl₃) δ : 2.86-2.96(4H, m), 3.65(2H, s), 5.02(2H, s), 6.93(1H, d, J=8.0Hz), 6.99(1H, dd, J=7.6,7.8Hz), 7.32(1H, dd, J=7.6,8.0Hz), 7.61(1H, d, J=7.8Hz).
Mass (FAB, m/z): 353(M+1), 355.

### Preparation example 21: Propionamidine

The above-titled product was obtained in the same method as in Preparation example 7 using propionitrile instead of 4-chlorophenoxyacetonitrile.
¹H-NMR (CD₃OD) δ : 1.28(3H, t, J=7.8Hz), 2.48(2H, q, J=7.8Hz).
Mass (EI, m/z): 72(M⁺).

### Example 57: 2-Ethyl-3,5,7,8-tetrahydrothiopyrano[4,3-d]pyrimidin-4-one

The above-titled product was obtained in the same method as in Example 1 except that propionamidine obtained in Preparation example 21 was used instead of 4-trifluoromethylbenzamidine.
¹H-NMR (10% CD₃OD/CDCl₃) δ : 1.33(3H, t, J=7.8Hz), 2.67(2H, q, J=7.8Hz), 2.86-2.96(4H, m), 3.62(2H, s).
Mass (EI, m/z): 196(M⁺).

### Preparation example 22: Fluoroacetamidine

The above-titled product was obtained in the same method as in Preparation example 7 using fluoroacetonitrile instead of 4-chlorophenoxyacetonitrile.
¹H-NMR (DMSO-d₆) δ : 5.31(2H, d, J=45.4Hz), 8.52(3H, brs).
Mass (EI, m/z): 76(M⁺).

### Example 58: 2-Fluoromethyl-3,5,7,8-tetrahydrothiopyrano[4,3-d]pyrimidin-4-one

The above-titled product was obtained in the same method as in Example 1 except that fluoroacetamidine obtained in Preparation example 22 was used instead of 4-trifluoromethylbenzamidine.
¹H-NMR (CDCl₃) δ : 2.86-2.95(4H, m), 3.64(2H, s), 5.30(2H, d, J=46.2Hz), 10.7(1H,brs).
Mass (EI, m/z): 200(M⁺).

### Preparation example 23: Chloroacetamidine

The above-titled product was obtained in the same method as in Preparation example 7 except that chloroacetonitrile was used instead of 4-chlorophenoxy-acetonitrile.
¹H-NMR (DMSO-d₆) δ : 4.44(2H, s), 9.36(3H, brs).
Mass (EI, m/z): 92(M⁺), 94.

### Example 59: 2-Chloromethyl-3,5,7,8-tetrahydrothiopyrano[4,3-d]pyrimidin-4-one

The above-titled product was obtained in the same method as in Example 1 except that chloroacetamidine obtained in Preparation example 23 was used instead of 4-trifluoromethylbenzamidine.
¹H-NMR (CDCl₃) δ : 2.86-2.96(4H, m), 3.63(2H, s), 4.46(2H, s).
Mass (EI, m/z): 216(M⁺), 218

### Example 60: 8-Benzylidene-2-methyl-3,5,7,8-tetrahydrothiopyrano[4,3-d]-pyrimidin-4-one

The product was obtained in the same method as in Example 34 except that benzaldehyde was used instead of propionaldehyde.
¹H-NMR (CDCl₃) δ : 2.50(3H, s), 3.76(2H, s), 3.79(2H, s), 7.28-7.44(5H, m), 8.08(1H, s).
Mass (EI, m/z): 270(M⁺).

### Example 61: 2-Methyl-8-(thiophen-3-ylmethylene)-3,5,7,8-tetrahydrothiopyrano-[4,3-d]pyrimidin-4-one

The product was obtained in the same method as in Example 34 except that 3-thiophenealdehyde was used instead of propionaldehyde.
¹H-NMR (CDCl₃) δ : 2.48(3H, s), 3.77(2H, s), 3.85(2H, s), 7.21(1H, d, J=3.2Hz), 7.36-7.39(1H, m), 8.03(1H, s).
Mass (EI, m/z): 276(M⁺).

### Example 62: 8-(Furan-2-ylmethylene)-2-methyl-3,5,7,8-tetrahydrothiopyrano-[4,3-d]pyrimidin-4-one

The product was obtained in the same method as in Example 34 except that 2-furaldehyde was used instead of propionaldehyde.
¹H-NMR (CDCl₃) δ: 2.47(3H, s), 3.77(2H, s), 4.09(2H, s), 6.50(1H, dd, J=1.9, 3.4Hz), 6.61(1H, d, J=3.4Hz), 7.54(1H, d, J=1.9Hz), 7.80(1H, s).
Mass (EI, m/z): 260(M⁺).

### Example 63: 2-Methyl-8-phenethylidene-3,5,7,8-tetrahydrothiopyrano[4,3-d] pyrimidin-4-one

The product was obtained in the same method as in Example 34 except that phenylacetaldehyde was used instead of propionaldehyde.
¹H-NMR (CDCl₃) δ : 2.41(3H, s), 3.62(2H, s), 3.64(2H, s), 3.74(2H, s), 7.20-7.35(5H, m).
Mass (EI, m/z): 284(M⁺).

### Example 64: 2-Methyl-8-(3-phenylpropylidene)-3,5,7,8-tetrahydrothiopyrano[4,3-d]-pyrimidin-4-one

The product was obtained in the same method as in Example 34 except that 3-phenylpropionaldehyde was used instead of propionaldehyde.
¹H-NMR (CDCl₃) δ : 2.44(3H, s), 2.55-2.64(2H, m), 2.83(2H, dd, J=7.3, 8.3Hz), 3.41(2H, s), 3.69(2H, s), 7.12-7.33(6H, m).
Mass (EI, m/z): 298(M⁺).

### Example 65: 2-Methyl-7,8-dihydro-3H,6H-thiopyrano[3,2-d]pyrimidin-4-one

The above-titled product was obtained in the same method as in Example 8 except that acetamidine was used instead of benzamidine.
¹H-NMR (CDCl₃) δ : 2.15-2.23(2H, m), 2.44(3H, s), 2.74(2H, t, J=6.3Hz), 2.95-3.00(2H, m).
Mass (EI, m/z): 182(M⁺).

### Formulation example 1

The compound according to the present invention tested in Pharmacological test example 1 (500 g) and D-mannitol (2.5 g) were dissolved in distilled water for injection to adjust the total amount to 50 ml. The solution was sterilized by filtration, and aliquots of 5 ml were filled in 10 ml vials, and then the vials were subjected to lyophilization and tightly sealed to give injections.

### Formulation example 2

The compound according to the present invention tested in Pharmacological test example 1 (25 g), lactose (32.5 g), corn starch (13.6 g) and crystalline cellulose (18 g) were mixed in a mortar and kneaded by adding an appropriate amount of water, and the kneaded product was dried in an air-flow dryer at 60°C for 60 minutes. The dried product was sized using a 30 mesh sieve, and the sized product was added with magnesium stearate (0.9 g) and mixed by using a V-type mixer for 10 minutes. The resulting mixture was compressed using a flat pounder having the diameter of 8 mm to give each 180 mg tablets.

### Test example 1: Measurement of PARP enzyme activity

Test method: The forebrain was collected from Wistar rats and homogenized. Nucleus obtained by centrifugation was extracted with 0.4 M NaCl and the resulting soluble fraction was used as a source of PARP enzyme source. The polyADP ribosylation was initiated by adding activated DNA derived from salmon sperm and the enzyme source to an enzyme reaction solution (100 mM Tris/HCl (pH 8.0), 20 mM MgCl₂, 1 mM DTT, 250 µM NAD, 0.1 µCi ³²P-NAD). Incubation was carried out at 37°C for 30 minutes, and then the reaction was stopped with 20%trichloroacetic acid. The acid-insoluble fraction produced above was adsorbed to a GF/B filter, and the filter was washed several times, and then radioactivity on the filter was measured by using a liquid scintillation counter. The PARP activity was measured by subtracting radioactivity as a blank value of a sample not added with the enzyme source. The 50% enzyme inhibitory value (IC₅₀ value) of each compound was calculated using values of the PARP activity of the compound at 6 different concentrations.

**Table 1**

| Example compound | PARP inhibitory activity IC₅₀ (µM) |
|---|---|
| Example 2 | 0.21 |
| Example 3 | 1.46 |
| Example 4 | 0.41 |
| Example 20 | 0.25 |
| Example 21(A) | 0.35 |
| Example 39 | 0.44 |

### Test example 2: Toxicity test example by single administration

The compound tested in Test example 1 was intraperitoneally administered to ddY male mice (6 weeks old, average body weight of about 30 to 40 g). As a result, at doses up to 200 mg/kg, no significant symptom was observed, which revealed low toxicity of the test compound.

### Test example 3: Toxicity test by continuous administration

The compound tested in Test example 1 was intravenously and continuously administered to Wistar male rats (12 weeks old, average body weight of about 200 to 300 g) over 4 hours. As a result, at doses up to 30 mg/kg, no effect on circulatory parameters such as blood pressure, electrocardiogram, and heart rate was observed during administration period. In addition, the compound had absolutely no effect on oxygen partial pressure and carbon dioxide partial pressure in blood, as well as on hydrogen ion concentration in blood.

From these results, it was revealed that the pyrimidine derivative of the present had potent PARP inhibitory activity.

### Industrial Applicability

The compounds represented by the formula (I) are useful as a new class of PARP inhibitor, and particularly useful as an active ingredient of a therapeutic and/or preventive medicament for a disease in which PARP is involved. In addition, the novel compounds provided by the present invention are useful as an active ingredient of medicaments.

## Claims

1. A medicament for therapeutic and/or preventive treatment of a brain disease, which comprises a compound represented by the following general formula (I) or a pharmaceutically acceptable salt thereof as an active ingredient: 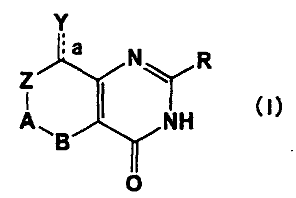 wherein R represents hydrogen atom, a C₁-C₈ alkyl group, a substituted C₁-C₈ alkyl group, an aryl group, a substituted aryl group, an aryl(C₁-C₈)alkyl group, a substituted aryl(C₁-C₈)alkyl group, hydroxyl group, mercapto group, a C₁-C₈ alkylmercapto group, amino group, a C₁-C₈ alkylamino group, a dialkylamino group, NHCOR¹ (R¹ represents hydrogen atom, a C₁-C₈ alkyl group, an aryl group, a substituted aryl group, an aryl(C₁-C₈)alkyl group, or a substituted aryl(C₁-C₈)alkyl group), a C₁-C₈ alkoxyl group, phenoxy group, a substituted phenoxy group, a heterocyclic group, or a substituted heterocyclic group;
Y represents hydrogen atom or -C(R²)R³ (R² and R³ are the same or different and represent hydrogen atom, a C₁-C₈ alkyl group, a C₁-C₈ alko(C₁-C₈)alkyl group, a hydroxy(C₁-C₈)alkyl group, an aryl(C₁-C₈)alkyl group, an aryl group, or a heterocyclic group);
symbol "a" represents single bond when Y represents hydrogen atom, or represents double bond when Y represents -C(R²)R³;
-A-B- represents -CH₂-CH₂-, -S-CH₂-, -O-CH₂-, -CH₂-S-, -CH₂-O-, -SO-CH₂-, -CH₂-SO-, -SO₂-CH₂-, or -CH₂-SO₂-; and
Z represents -CH₂- or single bond.

2. The medicament according to claim 1, wherein the brain disease is acute phase stroke.

3. The medicament according to claim 1, wherein the brain disease is Alzheimer's disease, Parkinson's disease, or Huntington's chorea.

4. A medicament having anti-inflammatory activity which comprises the compound represented by the general formula (I) or a pharmaceutically acceptable salt thereof according to claim 1 as an active ingredient.

5. The medicament according to any one of claims 1 to 4, which is in a form of a pharmaceutical composition comprising the compound represented by the general formula (I) or a pharmaceutically acceptable salt thereof according to claim 1 as an active ingredient and a pharmaceutical additive.

6. A poly(ADP-ribose)polymerase inhibitor which comprises the compound represented by the general formula (I) or a pharmaceutically acceptable salt thereof according to claim 1.

7. A use of the compound represented by the general formula (I) or a pharmaceutically acceptable salt thereof according to claim 1 for manufacture of the medicament according to any one of claims 1 to 5.

8. A method for therapeutic and/or preventive treatment of a brain disease, which comprises the step of administering a therapeutically and/or preventively effective amount of the compound represented by the general formula (I) or a pharmaceutically acceptable salt thereof according to claim 1 to a mammal including a human.

9. A method for therapeutic and/or preventive treatment of an inflammatory disease, which comprises the step of administering a therapeutically and/or preventively effective amount of the compound represented by the general formula (I) or a pharmaceutically acceptable salt thereof according to claim 1 to a mammal including a human.

10. A compound represented by the following general formula (I) or a salt thereof: 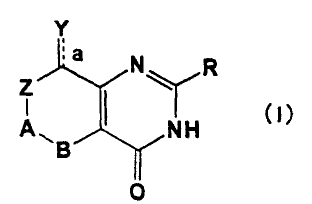 wherein R represents hydrogen atom, a C₁-C₈ alkyl group, a substituted C₁-C₈ alkyl group, an aryl group, a substituted aryl group, an aryl(C₁-C₈)alkyl group, a substituted aryl(C₁-C₈)alkyl group, hydroxyl group, mercapto group, a C₁-C₈ alkylmercapto group, amino group, a C₁-C₈ alkylamino group, a dialkylamino group, NHCOR¹ (R¹ represents hydrogen atom, a C₁-C₈ alkyl group, an aryl group, a substituted aryl group, an aryl(C₁-C₈)alkyl group, or a substituted aryl(C₁-C₈)alkyl group), a C₁-C₈ alkoxyl group, phenoxy group, a substituted phenoxy group, a heterocyclic group, or a substituted heterocyclic group;
Y represents hydrogen atom or -C(R²)R³ (R² and R³ are the same or different and represent hydrogen atom, a C₁-C₈ alkyl group, a Ci-Cs alkoxy(C₁-C₈)alkyl group, a hydrosy(C₁-C₈)alkyl group, an aryl(C₁-C₈)alkyl group, an aryl group, or a heterocyclic group);
symbol "a" represents single bond when Y represents hydrogen atom, or represents double bond when Y represents -C(R²)R³;
-A-B- represents -CH₂-CH₂-, -S-CH₂-, -O-CH₂-, -CH₂-S-, -CH₂-O-, -SO-CH₂-, -CE₂-SO-, -SO₂-CH₂-, or -CH₂-SO₂-; and
Z represents -CH₂- or single bond;
provided that the compound wherein:
R is hydrogen atom, a C₁-C₈ alkyl group, an aryl group, a methyl-substituted aryl group, an aryl(C₁-C₈)alkyl group, hydroxyl group, mercapto group, a C₁-C₈ alkylmercapto group, or amino group;
Y is hydrogen atom;
symbol "a" is single bond;
-A-B- is -CH₂-CH₂-, -S-CH₂-, -O-CH₂-, -CH₂-S-, -SO-CH₂-, or -SO₂-CH₂-, and
Z is -CH₂-
is excluded.

11. The compound or a salt thereof according to claim 10, wherein R is hydrogen atom, a C₁-C₈ alkyl group, a substituted C₁-C₈ alkyl group, an aryl group, a substituted aryl group, an aryl(C₁-C₈)alkyl group, a substituted aryl(C₁-C₈)alkyl group, a C₁-C₈ alkoxyl group, a heterocyclic group, or a substituted heterocyclic group; -A-B- is -CH₂-CH₂-, -S-CH₂-, -O-CH₂-, -CH₂-S-, -SO-CH₂-, or -SO₂-CH₂-.

12. A medicament which comprises the compound according to claim 10 or 11, or a pharmaceutically acceptable salt thereof as an active ingredient.

13. The medicament according to claim 12, which is used for therapeutic and/or preventive treatment of a brain disease.

14. The medicament according to claim 12, which has anti-inflammatory activity.

15. A poly(ADP-ribose)polymerase inhibitor which comprises the compound according to claim 10 or 11 or a pharmaceutically acceptable salt thereof.
